# EUROPEAN PATENT APPLICATION

(11) **EP 3 702 468 A1**
(43) Date of publication of application: **02.09.2020**
(21) Application number: 19160379.4
(22) Date of filing: 01.03.2019
(51) Int. Cl.: C12P 19/02, C12P 19/04, C12P 19/18, C12N 1/20, A61K 31/7004, A61K 31/7016, A61K 31/702

(54) **FERMENTATIVE PRODUCTION OF CARBOHYDRATES BY MICROBIAL CELLS UTILIZING A MIXED FEEDSTOCK**

(71) Applicant: Jennewein Biotechnologie GmbH, 53619 Rheinbreitbach (DE)
(72) Inventor: JENNEWEIN, Stefan, 53605 Bad Honnef (DE); WARTENBERG, Dirk, 55435 Gau-Algesheim (DE)

(57) **Abstract**

Disclosed are genetically engineered microbial cells for the production of a carbohydrate of interest, wherein the microbial cells possess an increased intracellular availability of at least one sugar phosphate, and produce the carbohydrate of interest when being cultivated in a culture medium containing a mixed monosaccharide feedstock as main carbon and energy source. Also disclosed is a fermentative method of producing a carbohydrate of interest by cultivating said genetically engineered microbial cell in the presence of a mixed monosaccharide feedstock as main carbon and energy source.

SEQUENCE LISTING

<110>  Jennewein Biotechnologie GmbH

<120>  Fermentative production of carbohydrates by microbial cells
       utilizing a mixed feedstock

<130>  P 1901 EP

<160>  16

<170>  PatentIn version 3.5

<210>  1
<211>  1395
<212>  DNA
<213>  Escherichia coli

<400>  1
atgcctgacg ctaaaaaaca ggggcggtca aacaaggcaa tgacgttttt cgtctgcttc      60

cttgccgctc tggcgggatt actctttggc ctggatatcg gtgtaattgc tggcgcactg     120

ccgtttattg cagatgaatt ccagattact tcgcacacgc aagaatgggt cgtaagctcc     180

atgatgttcg gtgcggcagt cggtgcggtg ggcagcggct ggctctcctt taaactcggg     240

cgcaaaaaga gcctgatgat cggcgcaatt ttgtttgttg ccggttcgct gttctctgcg     300

gctgcgccaa acgttgaagt actgattctt tcccgcgttc tactggggct ggcggtgggt     360

gtggcctctt ataccgcacc gctgtacctc tctgaaattg cgccggaaaa aattcgtggc     420

agtatgatct cgatgtatca gttgatgatc actatcggga tcctcggtgc ttatctttct     480

gataccgcct tcagctacac cggtgcatgg cgctggatgc tgggtgtgat tatcatcccg     540

gcaattttgc tgctgattgg tgtcttcttc ctgccagaca gcccacgttg gtttgccgcc     600

aaacgccgtt ttgttgatgc cgaacgcgtg ctgctacgcc tgcgtgacac cagcgcggaa     660

gcgaaacgcg aactggatga aatccgtgaa agtttgcagg ttaaacagag tggctgggcg     720

ctgtttaaag agaacagcaa cttccgccgc gcggtgttcc ttggcgtact gttgcaggta     780

atgcagcaat tcaccgggat gaacgtcatc atgtattacg cgccgaaaat cttcgaactg     840

gcgggttata ccaacactac cgagcaaatg tgggggaccg tgattgtcgg cctgaccaac     900

gtacttgcca cctttatcgc aatcggcctt gttgaccgct ggggacgtaa accaacgcta     960

acgctgggct tcctggtgat ggctgctggc atgggcgtac tcggtacaat gatgcatatc    1020

ggtattcact ctccgtcggc gcagtatttc gccatcgcca tgctgctgat gtttattgtc    1080

ggttttgcca tgagtgccgg tccgctgatt tgggtactgt gctccgaaat tcagccgctg    1140

aaaggccgcg attttggcat cacctgctcc actgccacca actggattgc caacatgatc    1200

gttggcgcaa cgttcctgac catgctcaac acgctgggta acgccaacac cttctgggtg    1260

tatgcggctc tgaacgtact gtttatcctg ctgacattgt ggctggtacc ggaaaccaaa    1320

cacgtttcgc tggaacatat tgaacgtaat ctgatgaaag gtcgtaaact gcgcgaaata 1380

ggcgctcacg attaa 1395

<210> 2
<211> 1422
<212> DNA
<213> Zymomonas mobilis

<400> 2
   atgagttctg aaagtagtca gggtctagtc acgcgactag ccctaatcgc tgctataggc 60
   ggcttgcttt tcggttacga ttcagcggtt atcgctgcaa tcggtacacc ggttgatatc 120
   cattttattg cccctcgtca cctgtctgct acggctgcgg cttccctttc tgggatggtc 180
   gttgttgctg ttttggtcgg ttgtgttacc ggttctttgc tgtctggctg gattggtatt 240
   cgcttcggtc gtcgcggcgg attgttgatg agttccattt gtttcgtcgc cgccggtttt 300
   ggtgctgcgt taaccgaaaa attatttgga accggtggtt cggctttaca aattttttgc 360
   tttttccggt ttcttgccgg tttaggtatc ggtgtcgttt caaccttgac cccaacctat 420
   attgctgaaa ttcgtccgcc agacaaacgt ggtcagatgg tttctggtca gcagatggcc 480
   attgtgacgg gtgctttaac cggttatatc tttacctggt tactggctca tttcggttct 540
   atcgattggg ttaatgccag tggttggtgc tggtctccgg cttcagaagg cctgatcggt 600
   attgccttct tattgctgct gttaaccgca ccggatacgc cgcattggtt ggtgatgaag 660
   ggacgtcatt ccgaggctag caaaatcctt gctcgtctgg aaccgcaagc cgatcctaat 720
   ctgacgattc aaaagattaa agctggcttt gataaagcca tggacaaaag cagcgcaggt 780
   ttgtttgctt ttggtatcac cgttgttttt gccggtgtat ccgttgctgc cttccagcag 840
   ttagtcggta ttaacgccgt gctgtattat gcaccgcaga tgttccagaa tttaggtttt 900
   ggagctgata cggcattatt gcagaccatc tctatcggtg ttgtgaactt catcttcacc 960
   atgattgctt cccgtgttgt tgaccgcttc ggccgtaaac ctctgcttat ttggggtgct 1020
   ctcggtatgg ctgcaatgat ggctgtttta ggctgctgtt tctggttcaa agtcggtggt 1080
   gttttgcctt tggcttctgt gcttctttat attgcagtct ttggtatgtc atggggccct 1140
   gtctgctggg ttgttctgtc agaaatgttc ccgagttcca tcaagggcgc agctatgcct 1200
   atcgctgtta ccggacaatg gttagctaat atcttggtta acttcctgtt taaggttgcc 1260
   gatggttctc cagcattgaa tcagactttc aaccacggtt tctcctatct cgttttcgca 1320
   gcattaagta tcttaggtgg cttgattgtt gctcgcttcg tgccggaaac caaaggtcgg 1380
   agcctggatg aaatcgagga gatgtggcgc tcccagaagt ag 1422

<210> 3
<211> 1248
<212> DNA

<213>  Escherichia coli

<400>  3
atggcactga atattccatt cagaaatgcg tactatcgtt ttgcatccag ttactcattt      60

ctctttttta tttcctggtc gctgtggtgg tcgttatacg ctatttggct gaaaggacat      120

ctagggttga cagggacgga attaggtaca ctttattcgg tcaaccagtt taccagcatt      180

ctatttatga tgttctacgg catcgttcag gataaactcg gtctgaagaa accgctcatc      240

tggtgtatga gtttcatcct ggtcttgacc ggaccgttta tgatttacgt ttatgaaccg      300

ttactgcaaa gcaatttttc tgtaggtcta attctggggg cgctattttt tggcttgggg      360

tatctggcgg gatgcggttt gcttgatagc ttcaccgaaa aaatggcgcg aaattttcat      420

ttcgaatatg gaacagcgcg cgcctgggga tcttttggct atgctattgg cgcgttcttt      480

gccggcatat tttttagtat cagtccccat atcaacttct ggttggtctc gctatttggc      540

gctgtattta tgatgatcaa catgcgtttt aaagataagg atcaccagtg cgtagcggca      600

gatgcgggag gggtaaaaaa agaggatttt atcgcagttt tcaaggatcg aaacttctgg      660

gttttcgtca tatttattgt ggggacgtgg tctttctata acatttttga tcaacaactt      720

tttcctgtct tttattcagg tttattcgaa tcacacgatg taggaacgcg cctgtatggt      780

tatctcaact cattccaggt ggtactcgaa gcgctgtgca tggcgattat tcctttcttt      840

gtgaatcggg tagggccaaa aaatgcatta cttatcggag ttgtgattat ggcgttgcgt      900

atcctttcct gcgcgctgtt cgttaacccc tggattattt cattagtgaa gttgttacat      960

gccattgagg ttccactttg tgtcatatcc gtcttcaaat acagcgtggc aaactttgat     1020

aagcgcctgt cgtcgacgat ctttctgatt ggttttcaaa ttgccagttc gcttgggatt     1080

gtgctgcttt caacgccgac tgggatactc tttgaccacg caggctacca gacagttttc     1140

ttcgcaattt cgggtattgt ctgcctgatg ttgctatttg gcattttctt cttgagtaaa     1200

aaacgcgagc aaatagttat ggaaacgcct gtaccttcag caatatag                  1248

<210>  4
<211>  735
<212>  DNA
<213>  Leuconostoc pseudomesenteroides

<400>  4
atggcacaaa acgcgcaaca tcataatccg cgaagcattt caatgagcaa atcacttatg      60

ttttttgcca tctcattgat tttaaatgcg atgggaaatg ttttgacgct cgtcacagct      120

tcacatataa aacccgcttt ttttggatca gcttattgga ctgctgcaga ggctaatcta      180

ggtcaagctt tattaggaaa taattcactt gtcttgtttt gggcattttt agttcttggc      240

atgcttattt cattcctgaa tgcgttatta atgaaaaagt tagattggca tcgtatcatt      300

ggtaatttct tgtttatgtt accattttca atttttattc aatggttttc aaatattttc      360

aatcaaatta tgccaaatgc taattcagtg gcagcaactg tgttatatac agtaattaat 420

tttattggtg ttggcttgat cgccgttgct atttcgattt atcaacgtgt gaatttagtg 480

ttacaccctg ccgatgattt gatgcaaatt ttacgtttca aatattttca cgggtcggct 540

ttcaaggcta tgtgggcgtc ctatattccg ccaacgattt ttgcaattat tgcctttgtg 600

atcactttcc ctaatttgta taatttcggg ttaggaatta tttttgcatt cttattccag 660

ggtggcatca caggaatcgc ggacaagtac gtctttaaga atttaaagca tcaggcaata 720

gatgttggta attaa 735

<210> 5
<211> 1254
<212> DNA
<213> Escherichia coli

<400> 5
atgtactatt taaaaaacac aaacttttgg atgttcggtt tattcttttt cttttacttt 60

tttatcatgg gagcctactt cccgtttttc ccgatttggc tacatgacat caaccatatc 120

agcaaaagtg atacgggtat tatttttgcc gctatttctc tgttctcgct attattccaa 180

ccgctgtttg gtctgctttc tgacaaactc gggctgcgca aatacctgct gtggattatt 240

accggcatgt tagtgatgtt tgcgccgttc tttattttta tcttcgggcc actgttacaa 300

tacaacattt tagtaggatc gattgttggt ggtatttatc taggcttttg ttttaacgcc 360

ggtgcgccag cagtagaggc atttattgag aaagtcagcc gtcgcagtaa tttcgaattt 420

ggtcgcgcgc ggatgtttgg ctgtgttggc tgggcgctgt gtgcctcgat tgtcggcatc 480

atgttcacca tcaataatca gtttgttttc tggctgggct ctggctgtgc actcatcctc 540

gccgttttac tctttttcgc caaaacggat gcgccctctt ctgccacggt tgccaatgcg 600

gtaggtgcca accattcggc atttagcctt aagctggcac tggaactgtt cagacagcca 660

aaactgtggt ttttgtcact gtatgttatt ggcgtttcct gcacctacga tgtttttgac 720

caacagtttg ctaatttctt tacttcgttc tttgctaccg gtgaacaggg tacgcgggta 780

tttggctacg taacgacaat gggcgaatta cttaacgcct cgattatgtt ctttgcgcca 840

ctgatcatta atcgcatcgg tgggaaaaac gccctgctgc tggctggcac tattatgtct 900

gtacgtatta ttggctcatc gttcgccacc tcagcgctgg aagtggttat tctgaaaacg 960

ctgcatatgt ttgaagtacc gttcctgctg gtgggctgct ttaaatatat taccagccag 1020

tttgaagtgc gtttttcagc gacgatttat ctggtctgtt tctgcttctt taagcaactg 1080

gcgatgattt ttatgtctgt actggcgggc aatatgtatg aaagcatcgg tttccagggc 1140

gcttatctgg tgctgggtct ggtggcgctg ggcttcacct taatttccgt gttcacgctt 1200

agcggccccg gcccgctttc cctgctgcgt cgtcaggtga atgaagtcgc ttaa 1254

<210>  6
<211>  1317
<212>  DNA
<213>  Escherichia coli

<400>  6
atgggaaaca catcaataca aacgcagagt taccgtgcgg tagataaaga tgcagggcaa      60

agcagaagtt acattattcc attcgcgctg ctgtgctcac tgttttttct ttgggcggta      120

gccaataacc ttaacgacat tttattacct caattccagc aggcttttac gctgacaaat      180

ttccaggctg gcctgatcca atcggccttt tactttggtt atttcattat cccaatccct      240

gctgggatat tgatgaaaaa actcagttat aaagcaggga ttattaccgg gttattttta      300

tatgccttgg gtgctgcatt attctggccc gccgcagaaa taatgaacta caccttgttt      360

ttagttggcc tatttattat tgcagccgga ttaggttgtc tggaaactgc cgcaaaccct      420

tttgttacgg tattagggcc ggaaagtagt ggtcacttcc gcttaaatct tgcgcaaaca      480

tttaactcgt ttggcgcaat tatcgcggtt gtctttgggc aaagtcttat tttgtctaac      540

gtgccacatc aatcgcaaga cgttctcgat aaaatgtctc cagagcaatt gagtgcgtat      600

aaacacagcc tggtattatc ggtacagaca ccttatatga tcatcgtggc tatcgtgtta      660

ctggtcgccc tgctgatcat gctgacgaaa ttcccggcat tgcagagtga taatcacagt      720

gacgccaaac aaggatcgtt ctccgcatcg ctttctcgcc tggcgcgtat tcgccactgg      780

cgctgggcgg tattagcgca attctgctat gtcggcgcac aaacggcctg ctggagctat      840

ttgattcgct acgctgtaga agaaattcca ggtatgactg caggctttgc cgctaactat      900

ttaaccggaa ccatggtgtg cttctttatt ggtcgtttca ccggtacctg gctcatcagt      960

cgcttcgcac cacacaaagt cctggccgcc tacgcattaa tcgctatggc actgtgcctg     1020

atctcagcct tcgctggcgg tcatgtgggc ttaatagccc tgactttatg cagcgccttt     1080

atgtcgattc agtacccaac aatcttctcg ctgggcatta agaatctcgg ccaggacacc     1140

aaatatggtt cgtccttcat cgttatgacc attattggcg gcggtattgt cactccggtc     1200

atgggttttg tcagtgacgc ggcgggcaac atccccactg ctgaactgat ccccgcactc     1260

tgcttcgcgg tcatctttat ctttgcccgt ttccgttctc aaacggcaac taactga        1317

<210>  7
<211>  1491
<212>  DNA
<213>  Escherichia coli

<400>  7
atgagtacta caacccagaa tatcccgtgg tatcgccatc tcaaccgtgc acaatggcgc      60

gcattttccg ctgcctggtt gggatatctg cttgacggtt ttgatttcgt tttaatcgcc      120

ctggtactca ccgaagtaca aggtgaattc gggctgacga cggtgcaggc ggcaagtctg 180

atctctgcag cctttatctc tcgctggttc ggcggcctga tgctcggcgc tatgggtgac 240

cgctacgggc gtcgtctggc aatggtcacc agcatcgttc tcttctcggc cgggacgctg 300

gcctgcggct ttgcgccagg ctacatcacc atgtttatcg ctcgtctggt catcggcatg 360

gggatggcgg gtgaatacgg ttccagcgcc acctatgtca ttgaaagctg gccaaaacat 420

ctgcgtaaca aagccagtgg ttttttgatt tcaggcttct ctgtgggggc cgtcgttgcc 480

gctcaggtct atagcctggt ggttccggtc tggggctggc gtgcgctgtt ctttatcggc 540

attttgccaa tcatctttgc tctctggctg cgtaaaaaca tcccggaagc ggaagactgg 600

aaagagaaac acgcaggtaa agcaccagta cgcacaatgg tggatattct ctaccgtggt 660

gaacatcgca ttgccaatat cgtaatgaca ctggcggcgg ctactgcgct gtggttctgc 720

ttcgccggta acctgcaaaa tgccgcgatc gtcgctgttc ttgggctgtt atgcgccgca 780

atctttatca gctttatggt gcagagtgca ggcaaacgct ggccaacggg cgtaatgctg 840

atggtggtcg tgttgtttgc tttcctctac tcatggccga ttcaggcgct gctgccaacg 900

tatctgaaaa ccgatctggc ttataacccg catactgtag ccaatgtgct gttctttagt 960

ggctttggcg cggcggtggg atgctgcgta ggtggcttcc tcggtgactg gctgggaacc 1020

cgcaaagcgt acgtttgtag cctgctggcc tcgcagctgc tgattattcc ggtatttgcg 1080

attggcggcg caaacgtctg ggtgctcggt ctgttactgt tcttccagca aatgcttgga 1140

caagggatcg ccgggatctt accaaaactg attggcggtt atttcgatac cgaccagcgt 1200

gcagcgggcc tgggctttac ctacaacgtt ggcgcattgg gcggtgcact ggccccaatc 1260

atcggcgcgt tgatcgctca acgtctggat ctgggtactg cgctggcatc gctctcgttc 1320

agtctgacgt tcgtggtgat cctgctgatt gggctggata tgccttctcg cgttcagcgt 1380

tggttgcgcc cggaagcgtt gcgtactcat gacgctatcg acggtaaacc attcagcggt 1440

gccgtgccgt ttggcagcgc caaaaacgat ttagtcaaaa ccaaaagtta a 1491

<210> 8
<211> 1278
<212> DNA
<213> Xanthomonas campestris

<400> 8
atgactaccg ccaggcccgc aaatcccgtt gtctcgatcg ccatcgtcgg cgtgctgttt 60

ttcatcatcg gctttttcac ctggatcaac gggccgctga tcaccttcgt gcggctggcg 120

ttcgacctca acgaggtcaa tgcgttcctg gtgctgatgg tgttctacct gtcgtacttc 180

ttcctggcgc tgccctcgtc atggatcctc aagcgcaccg gcatgaaaaa aggcctggcg 240

ctgagtctgg tggtgatggc gctgggcgcc gcagcattcg ggcaattcgc tacgcaacgc 300

tggtatccgg gcgcactggc cggcatgttc gtgatcggta gcggcctggc gttgttgcag 360

accgcgatca acccatacat cagtattctc gggccaatcg aaagtgcggc gcggcgcatc 420

gcgctgatgg gcatctgtaa caagattgcc ggcatcctgg cgccgatcct gatcggctcg 480

ctggtgctgc atggcatcgg cgatctctcc acgcaggtgg ccgcggccga tgcggcgacc 540

aagcagcaac tgctcaacgc gtttgccgcc aagatccatg caccatatct ggtgatgtcc 600

ggcgtgttgc tggtgctggc ggtgggcgtg ttgttctcgc cgctgcccga actcaaggcc 660

tccgaagcca atgccacgcc gggcagcggt ggcgcggcgc agaagtccag catcttccag 720

ttcccgcatc tgtggctggg cgtgttgtgc ctgttcgtgt atgtcggtgt ggaagtgatg 780

gccggcgatg ccatcggcac ttacggacac ggcttcaatt tgccgctgga cagcaccaag 840

ctgttcacct cctacacgct gggcgcaatg ttgctgggct atatcgccgg cctggtgctg 900

attccgcggg tgatttcgca ggcgcgctac ctgagcgtgt ctgcgctgct gggcgtgctg 960

ttctcgctgg gggcgctgtt cacccacggc tatgtgtcgg tggggttcgt ggccgcgctc 1020

ggttttgcca acgccatgat gtggccggcg atctttccgc tggccatccg cggcctgggc 1080

cggttcaccg agatcggttc ggcgttgctg gtgatgggca ttgccggcgg cgcgatcatt 1140

ccgcagctgt tcgccattct gaaacagcat tacgacttcc aggtggtgtt cgccgcgctg 1200

atggtgccgt gctacctgta catcctgttc tattcgctgc gcggccaccg tgtggggctg 1260

ccggctcaac cgaaatga 1278

<210> 9
<211> 1554
<212> DNA
<213> Bifidobacterium longum

<400> 9
atgacgacga caacggcatc acccgtatcg aaacagacgg catccgcggc gcaggaaacc 60

agcgcaaccg gtgcggcggc caccgcaatc gaaaccatcg aaaccggcgt ggccggagtg 120

gcgggcgcgg ccacaaacgc ggcagccaac gcaatcgagg acctcgaagc cgccgaatcg 180

cacggcttct ccacgcgctt cccgctcaac agcgcattca tcttcacctt cggcgcgctc 240

ggcggcatgc tgttcggttt cgacaccggc atcatctccg gcgcctcccc gcttatcgaa 300

tcggacttcg gcctgagcgt ctctcagacc ggcttcatca cctcctcggt gctgatcggc 360

tcgtgcgcag gcgcgctgtc gatcggcgca ctgtctgacc ggttcggccg caagaagctg 420

ctcatcgtct ccgcgctgct gttcctgctc ggctcaggcc tgtgcgcctc ctccaccgga 480

ttcgcgatga tggtgtgcgc ccgcatcatc ctgggtctcg ccgtcggcgc ggcctccgcc 540

ctgaccccgg cgtacttggc cgaactggcg ccgaaggagc gtcgcggctc actgtccacg 600

ctgttccagc tcatggtcac cttcggcatt ctgctggcct acgcctccaa cctcggattc 660

ctgaaccaca acctcttcgg catccgcgac tggcgctgga tgctcggttc ggcgctggtg 720

ccggccgcct tgttgctgct cggcggcctg ttgctgcccg aatccccgcg ttatctggtg 780

aacaagggcg acacccgcaa cgccttcaaa gtgcttacgc tgattcgcaa ggacgtggat 840

cagacccagg tgcagattga gctggacgaa atcaaggccg tggccgcaca ggacaccaag 900

ggtggtgtgc gcgaactgtt ccgtatcgct cgtccggcgc tggtggccgc catcggtatc 960

atgctgttcc agcagctcgt gggcatcaac tcggtgatct acttcctgcc gcaggtgttc 1020

atcaagggct tcggcttccc tgaaggcgac gcgatctggg tgtcggtggg catcggcgtg 1080

gtgaacttcg tgagcaccat cgtggccacg cttatcatgg atcgtttccc gcgcaagggc 1140

atgctgatct tcggttccat cgtgatgacc gtttcgctcg cggtgctcgc cgtgatgaac 1200

ttcgtgggcg acgtggccgt gctggcagtg ccgacgatga ttctcatcgc tttctatatc 1260

ctcggctttg cggtctcgtg gggcccgatc gcctgggtgc ttatcggcga gatcttcccg 1320

ctgagcgtgc gcggcatcgg ctcatccttc ggctcggcgg cgaactggct gggcaacttc 1380

atcgtctccc agttcttcct cgtgctgctc gatgcgttcg gcaacaatgt gggcggcccg 1440

ttcgcgattt tcggcgtgtt ctcggccctg tccatcccgt tcgtgctgcg cttggtgccc 1500

gagaccaagg gcaagtcgct ggaggaaatc gagaaggaaa tgaccaagcg ctag 1554

<210> 10
<211> 1206
<212> DNA
<213> Bacillus subtilis

<400> 10
atgttaagag ggacatattt atttggatat gctttctttt ttacagtagg tattatccat 60

atatcaacag ggagtttgac accattttta ttagaggctt ttaacaagac aacagatgat 120

atttcggtca taatcttctt ccagtttacc ggatttctaa gcggagtatt aatcgcacct 180

ttaatgatta agaaatacag tcattttagg acacttactt tagctttgac aataatgctt 240

gtagcgttaa gtatcttttt tctaaccaag gattggtatt atattattgt aatggctttt 300

ctcttaggat atggagcagg cacattagaa acgacagttg gttcatttgt tattgctaat 360

ttcgaaagta atgcagaaaa aatgagtaag ctggaagttc tctttggatt aggcgcttta 420

tctttcccat tattaattaa ttccttcata gatatcaata actggttttt accatattac 480

tgtatattca cctttttatt cgtcctattc gtagggtggt taattttctt gtctaagaac 540

cgagagtacg ctaagaatgc taaccaacaa gtgacctttc cagatggagg agcatttcaa 600

tactttatag gagatagaaa aaaatcaaag caattaggct tttttgtatt tttcgctttc 660

ctatatgctg gaattgaaac aaattttgcc aactttttac cttcaatcat gataaaccaa 720

gacaatgaac aaattagtct tataagtgtc tcctttttct gggtagggat catcatagga 780

agaatattga ttggtttcgt aagtagaagg cttgattttt ccaaatacct tctttttagc 840

tgtagttgtt taattgtttt gttgattgcc ttctcttata taagtaaccc aatacttcaa 900

ttgagtggta catttttgat tggcctaagt atagcgggga tatttcccat tgctttaaca 960

ctagcatcaa tcattattca gaagtacgtt gacgaagtta caagtttatt tattgcctcg 1020

gcaagtttcg gaggagcgat catctctttc ttaattggat ggagtttaaa ccaggatacg 1080

atcttattaa ccatgggaat atttacaact atggcggtca ttctagtagg tatttctgta 1140

aagattagga gaactaaaac agaagaccct atttcacttg aaaacaaagc atcaaaaaca 1200

cagtag 1206

<210> 11
<211> 1593
<212> DNA
<213> Vibrio parahaemolyticus

<400> 11
atggtcttcg ccatttatgt cgcaattatc attggggtcg gactttgggt atctcgtgat 60

aaaaaaggca ctcagaaaag tacggaagat tatttcttgg cgggaaaatc tttgccttgg 120

tgggctgtcg gtgcttcgct aattgctgca aatatttctg cggaacaatt tataggaatg 180

tctggttcag gctattcaat tggcttggct atcgcatctt atgaatggat gtcggcaata 240

acattgatta ttgttggtaa gtactttcta cctattttca ttgaaaaagg aatctatacc 300

attcctgaat ttgttgaaaa acgcttcaat aaaaaactaa aaacaatttt ggccgttttt 360

tggatttcct tgtacatttt tgtaaaccta acttcagtac tgtatttagg tggtttggct 420

ctcgaaacca ttttgggtat tccgttgatg tactcaattc taggtcttgc gctgtttgcg 480

ttggtgtact caatttatgg tggtttatcg gcagtagtat ggaccgatgt catccaagtg 540

ttcttcttag ttttgggtgg ttttatgact acctacatgg cagtgagctt tattggtggt 600

acggacggtt ggttcgctgg ggtgtctaaa atggtcgatg cagctcctgg ccactttgag 660

atgatcttgg atcaaagtaa tccacaatac atgaaccttc ctggtattgc cgtattaatt 720

ggtggtcttt gggtagcaaa cttatattac tggggcttta accagtacat tattcaaaga 780

acgcttgctg caaaatcagt atcggaagct cagaaaggta ttgtttttgc agcgtttttg 840

aaacttatcg ttccgtttct cgtggtattg ccaggtattg ccgcttacgt tattacttcg 900

gacccacaac taatggcaag ccttggtgat attgcagcaa caaaccttcc aagtgctgct 960

aatgcggata aagcataccc ttggctaact cagttcttgc ctgttggtgt taaaggtgtt 1020

gtttttgcgg ctcttgctgc tgcaattgtt tcttcactag catcaatgct taactcaaca 1080

gccactatct tcactatgga tatttacaaa gagtatatct ctcctgactc aggtgaccac 1140

aagttggtga atgttgggcg tactgcagct gtggtggcac taattattgc ttgcctaatt 1200

gccccaatgt taggtggtat tggccaagca ttccaataca tccaagaata tacaggttta 1260

gttagccctg gtattttggc tgtattctta cttggcttat tctggaagaa aacaaccagt 1320

aaaggggcta ttattggtgt agtagcatca ataccatttg ccttgttctt gaaatttatg 1380

ccactttcca tgccatttat ggatcaaatg ctatacacat tattgtttac aatggttgtt 1440

atcgcattta caagtttgag cacatcaatt aatgatgatg atcctaaagg tattagtgtt 1500

acatcatcga tgtttgtaac agatcgaagc tttaatatcg ctgcttacgg cataatgatt 1560

gttttggcag tgttatatac attgttctgg taa 1593

<210> 12
<211> 1476
<212> DNA
<213> Escherichia coli

<400> 12
atgaataccc agtataattc cagttatata ttttcgatta ccttagtcgc tacattaggt 60

ggtttattat ttggctacga caccgccgtt atttccggta ctgttgagtc actcaatacc 120

gtctttgttg ctccacaaaa cttaagtgaa tccgctgcca actccctgtt agggttttgc 180

gtggccagcg ctctgattgg ttgcatcatc ggcggtgccc tcggtggtta ttgcagtaac 240

cgcttcggtc gtcgtgattc acttaagatt gctgctgtcc tgttttttat ttctggtgta 300

ggttctgcct ggccagaact tggttttacc tctataaacc cggacaacac tgtgcctgtt 360

tatctggcag gttatgtccc ggaatttgtt atttatcgca ttattggcgg tattggcgtt 420

ggtttagcct caatgctctc gccaatgtat attgcggaac tggctccagc tcatattcgc 480

gggaaactgg tctcttttaa ccagtttgcg attattttcg ggcaactttt agtttactgc 540

gtaaactatt ttattgcccg ttccggtgat gccagctggc tgaatactga cggctggcgt 600

tatatgtttg cctcggaatg tatccctgca ctgctgttct taatgctgct gtataccgtg 660

ccagaaagtc ctcgctggct gatgtcgcgc ggcaagcaag aacaggcgga aggtatcctg 720

cgcaaaatta tgggcaacac gcttgcaact caggcagtac aggaaattaa acactccctg 780

gatcatggcc gcaaaaccgg tggtcgtctg ctgatgtttg gcgtgggcgt gattgtaatc 840

ggcgtaatgc tctccatctt ccagcaattt gtcggcatca atgtggtgct gtactacgcg 900

ccggaagtgt tcaaaacgct gggggccagc acggatatcg cgctgttgca gaccattatt 960

gtcggagtta tcaacctcac cttcaccgtt ctggcaatta tgacggtgga taaatttggt 1020

cgtaagccac tgcaaattat cggcgcactc ggaatggcaa tcggtatgtt tagcctcggt 1080

accgcgtttt acactcaggc accgggtatt gtggcgctac tgtcgatgct gttctatgtt 1140

gccgcctttg ccatgtcctg gggtccggta tgctgggtac tgctgtcgga aatcttcccg 1200

aatgctattc gtggtaaagc gctggcaatc gcggtggcgg cccagtggct ggcgaactac 1260

ttcgtctcct ggaccttccc gatgatggac aaaaactcct ggctggtggc ccatttccac 1320

aacggtttct cctactggat ttacggttgt atgggcgttc tggcagcact gtttatgtgg 1380

aaatttgtcc cggaaaccaa aggtaaaacc cttgaggagc tggaagcgct ctgggaaccg 1440

gaaacgaaga aaacacaaca aactgctacg ctgtaa 1476

<210> 13
<211> 1395
<212> DNA
<213> Bacillus subtilis

<400> 13
atgaagaata ctccaactca attagaacca aatgttcctg taacaagaag ccattcaatg 60

ggatttgtca ttttgatctc atgtgcggcg gggcttggcg gcttattgta tggctatgac 120

acggcagtga tttctggcgc catcggtttt ctgaaagatt tatacagcct gagtccgttt 180

atggagggac ttgtcatttc aagcattatg attggaggag ttgtgggcgt cgggatatcc 240

ggatttttaa gtgacagatt cggccggaga aaaattttaa tgacagccgc tttgttattt 300

gcgatatcag caatcgtttc agcgctttct caagacgtgt ccaccttaat cattgcaagg 360

attatcgggg ggctgggaat cgggatgggc tcatcgctct ctgttacgta tattacagaa 420

gcggcaccgc ccgctatacg cggaagttta tcttcgttat atcagctctt tacgatactg 480

ggtatttccg caacatactt tattaatcta gctgtgcagc ggtccggaac atacgaatgg 540

ggcgtgcaca ccggctggag atggatgctt gcttatggaa tggtgccatc cgtcattttt 600

ttccttgtcc tgctcgtcgt cccggaaagt ccgagatggc tggcgaaagc gggcaaaaca 660

aatgaagctt taaagatcct gacacgtatt aatggagaaa ctgttgcaaa agaagaatta 720

aagaacattg agaactcttt aaaaatagaa caaatggggt cgctctccca gctgtttaag 780

ccgggtctca gaaaggcgct tgtcattgga atcctgctgg cgctgtttaa ccaagtcatc 840

ggcatgaacg cgattactta ctacggcccg gaaatcttta aaatgatggg attcgggcaa 900

aacgccggat ttgtgacgac ttgtatcgtc ggggttgtag aagttatttt taccgttatt 960

gcggtgttgc tgattgataa agtcggacga aaaaaactga tgtccatcgg ttctgctttt 1020

atggctattt ttatgatttt aatcgggacg tcgttttatt ttgagttaac aagcgggatc 1080

atgatgatcg tccttatatt aggttttgtc gctgctttct gtgtctcggt cggaccgatc 1140

acatggatta tgatttctga aatcttcccg aaccatctgc gtgcgcgggc cgcgggcatt 1200

gcgaccatct ttttatgggg agcaaactgg gcgatcggac agtttgtgcc aatgatgatc 1260

gattctttcg ggctcgccta tacattttgg atctttgcgg tgattaacat cctttgtttc 1320

ctgtttgtcg ttacgatctg tccagaaacg aagaacaaat cgctcgagga aattgaaaag 1380

ctttggataa aatga 1395

<210> 14
<211> 4393
<212> DNA
<213> Klebsiella pneumoniae

<400> 14
atgtataaaa aacggaagtt agccattctt attgctttgc taaccggcac cgccgccgcc 60

catgggcaga cagacctgaa cagcattgaa gcgcgtctcg ccgccctgga aaaacgcctg 120

caggacgccg agacccgcgc cagcactgcc gaaagccgcg ccgcctcagc ggagcagaaa 180

gttcagcagt taacccagca gcagcagcaa acccaggcca ccacccagca ggtggccagg 240

cgcaccactc aactggaaga aaaagccgaa cggcccggcg gctttgagtt ccatggctat 300

gcgcgttccg gggtgatcat gaacgactcg gccgccagta ccaaatccgg cgcttatatg 360

acccccgccg gggagaccgg cggcgccatt ggtcgcctgg gcaaccaggc cgacacctat 420

gtggaaatga acctcgaaca taaacagacc ctggacaacg gggcgaccac ccgtttcaaa 480

gtgatggtgg ccgacggaca gaccacctat aacgactgga cggcaagcag cagcgacctg 540

aacgtacgcc aggcgttcgt cgagctgggc aatctgccga ccttcgaagg cccgttcaaa 600

ggctcgaccc tgtgggccgg gaaacgcttt gaccgcgaca acttcgacat ccactggatt 660

gactcggatg tggtgttcct cgccgggacc ggcggcggga tctacgacgt gaaatggaac 720

gacagcctgc gcagcaactt ctcgttatac ggccgcaact ttggcgatat cgccgacagc 780

agcaacagcg tgcagaacta tatcgtcagc atgaataact ttgccggccc ggtgcagatg 840

atggtcagcg ggatgcgggc gaaagataat gacgaccgcc aggacgcgaa cggcaatctg 900

gtgaaaggcg atgccgctaa caccggggtt catgccctgc tgggcctgca caatgagagc 960

ttctatggcc tgcgcgacgg gaccagcaaa acggccctgc tgtacggcca cgggctgggc 1020

gccgaggtta aaggcatcgg ctccgacggc gcgctgcgcc cgggggccaa tacctggcgc 1080

ttcgccagct atggcactac gccgctgagc gatcgctggt ttattgcccc ggccgtgctg 1140

gcgcagagca gtaaagatcg ttatgtcgat ggcgacagct atcagtgggc caccctcaac 1200

ctgcgtctga ttcaggaagt gacgcagaac ttcgccctcg cctgggaggg cagctatcag 1260

tacatggatc tgcagcctga aggctacaac gatcgccatg cggtcaatgg cagcttctac 1320

aagctgacct tcgccccgac cttcaaggtg ggcagcatcg gcgacttctt ctcgcggccg 1380

gagatccgct tctatacatc gtggatggac tggagcaaaa aactggacaa ctacgccaac 1440

gatgacgcgt taggcagcaa cggattcaaa tcgggcggcg aatggtcgtt cggtatgcaa 1500

atggagacct ggttctgacg gccaccgggg cgacagggta aataacacat aaatataagg 1560

ttcgcggcgc ctgccacggc tggcgccgcc cacgccatat catcatgcat ttagagggta 1620

ctatggattt tgaacagatt tcccgctcac tgcttcccct gctgggcggc aaggaaaata 1680

tcgccagcgc cgcgcactgc gccacccgcc tgcggctggt gctggtcgac gacgcgctcg 1740

ccgatcagca ggcgattggc aaaatcgacg gggtgaaagg ctgctttcgc aatgccggac 1800

agatgcagat catcttcggc accggggtgg tcaataaagt ctatgccgcc tttatccagg 1860

ccgcaggcat cagcgaatcg agcaaatccg aagccgccga cctggcggcg aaaaagctga 1920

acccgttcca gcgcatcgcc cgcctgctgt ccaacatctt cgtgccgatt attccggcca 1980

tcgtcgcctc cggcctgctg atgggcctgc tggggatggt gaaaacctac ggttgggtcg 2040

acccgagcaa cgctctctat atcatgctgg atatgtgcag ttcggcggcg tttatcattc 2100

tgccgatcct gatcggcttt accgccgccc gcgaatttgg cggtaacccc tatctgggcg 2160

cgaccctcgg cgggatcctc acccacccgg cgctgaccaa cgcctggggc gtcgccgccg 2220

gcttccacac catgaatttc ttcggcatcg aagtggcgat gatcggctac cagggcaccg 2280

tcttcccggt gctgctggcg gtgtggttta tgagcatggt cgagaagcgg ctgcgccgcg 2340

tgatccctga cgcgctggac ctgatcctca ctccgttcct gacggtgatt atctccggct 2400

ttatcgccct gctgctgatc ggcccggccg gtcgcgcgct cggcgacggc atttcgttta 2460

tcctcagcac gcttatcagc catgccggct ggctggcggg cctgctgttc ggcggcctct 2520

attcggtgat cgtcattacc ggtatccatc acagcttcca tgccatcgag gccggactgc 2580

tgggcaaccc atcgattggc gtcaacttcc tgctgccgat ctgggcgatg gccaacgtcg 2640

cccagggcgg cgcctgcttt gcggtgtggt ttaaaaccaa agatgccaaa ataaaagcca 2700

tcaccctgcc gtcggcgttt tcggcgatgc tggggatcac cgaggcggca atcttcggga 2760

ttaacctgcg ctttgtgaaa ccgtttatcg ccgcgctggt gggcggtgcc gccggcggcg 2820

cctgggtggt gtcgatgcac gtctacatga ccgcggtggg cctgacggcg atcccgggaa 2880

tggctatcgt gcaggccagc tcgctgctga actacattat cggaatggcg atcgccttcg 2940

ccgtggcctt cgcgctctct ctgacgctga aatacaaaac ggacgctgaa taatgtcatt 3000

accgtcacgt ctgcctgcga tcctgcaggc cgttatgcag ggccagccgc aggcgctggc 3060

cgacagccat tatccgcaat ggcatctggc gccggtcaac ggactgctga acgatcctaa 3120

cggcttttgc caggtcgccg ggcgttacca cctgttttat cagtggaacc cgctcgcctg 3180

cgaccatacc tataagtgct ggggacactg gagctctgcc gatctgctgc actggcggca 3240

cgaacctatc gccctgatgc cggatgaaga gtatgaccgc aacggctgct actctggcag 3300

cgcggtcgag ttcgagggtg ccctgactct gtgctacacc ggcaacgtga aattccccga 3360

cggcgggcgc accgcctggc aatgtctggc gaccgagaat gccgatggca ccttccgcaa 3420

gctggggccg gtgctgccgc tgccagaagg ctataccggc catgtgcgcg accctaaagt 3480

gtggcggcag gacgggcgct ggtacatggt tcttggggcg caggatgtgc aacagcgcgg 3540

caaagtgctg ctgtttaccg ccagcgacct gcgggagtgg cgcctggtgg gcgagatcgc 3600

cgggcacgac gtgaacggcc tggcgaacgc cggctacatg tgggagtgcc cggatctctt 3660

tccgctggcg gacacccacc tgctgatctg ctgcccgcag gggctggccc gcgaagcgca 3720

gcgctttctc aatacctatc cggcggtgtg gatggcaggc cgcttcgacg ccgaacgcgg 3780

gatcttcgac cacggcccgc tgcacgagct ggacagcgga tttgagttct acgcgccgca 3840

gaccatgcag gccgacgatg gccgccgcct gctggtcggc tggatgggcg tccccgacgg 3900

ggacgagatg catcagccca cccgcgcgca gggctggatc catcagatga cctgcgtgcg 3960

tgagctggag tggcaggctg gcactctgta tcagcgtccg ctgcgcgagc tggtcgccct 4020

gcgcggggaa gcccagggct ggtgcggaca gaccctgccc ctcgccccga tggagctggc 4080

ctttgacctt tcccccgaca gcacgctggg gctggacttt gccggcgccc tgcagctcac 4140

cgtcaatcgc gacggcctac gtctgtcgcg ccgcggcctg cagacggcag agatgcatca 4200

ccgctactgg cgcggcgagg cgcgacgcct gcggatcttt atcgaccgct ccagcgtgga 4260

gattttcatc aacgatggcg agggggtgat gagcagccgc ttctttccgg gctatccggg 4320

gcagctcatc ttcagcggtg cgacgccggt ggcattctgc cgctggctgc tgcggccatg 4380

catggtagaa taa 4393

<210> 15
<211> 4423
<212> DNA
<213> Salmonella typhimurium

<400> 15
atgtacagaa aaagcacact tgcgatgctt atcgctttgc taaccagcgc tgcctcagcc 60

catgcgcaaa cggatataag caccattgaa gcccgactca acgcgctgga aaaacgcctg 120

caggaggcag aaaacagggc gcaaacggcg gaaaaccgcg ccggggcggc ggagaaaaaa 180

gttcagcaac tcaccgcgca gcagcaaaaa aaccagaact cgactcagga agtggctcag 240

cgtaccgcca gacttgagaa aaaagccgat gacaaaagcg gatttgagtt tcacggttac 300

gcccgctccg gcgtgataat gaatgattcc ggcgccagca ccaaatccgg agcctacata 360

acgccggcag gtgaaaccgg cggagctatc ggccgtctgg gaaaccaggc cgatacctat 420

gttgaaatga atcttgaaca taagcagacc ctggataatg gggccacgac ccgctttaag 480

gtgatggtcg ccgacgggca aacctcttat aacgactgga ctgcaagcac cagcgatctg 540

aacgttcgtc aggcctttgt cgaattgggt aacctgccga cgttcgctgg gccatttaag 600

ggctccaccc tgtgggccgg gaaacgtttc gaccgcgaca atttcgatat tcactggatt 660

gactctgatg tcgtgttcct cgccggtacc ggtggtggta tctatgacgt gaagtggaac 720

gacggcctgc ggagtaattt ctccctgtac gggcgtaact tcggcgacat tgatgattcc 780

agcaacagcg tgcagaacta tatcctcacc atgaatcact tcgcaggtcc gctgcagatg 840

atggtcagcg gtctgcgggc gaaggataac gacgagcgta aagatagcaa cggcaatctg 900

gcaaaaggcg atgcggcaaa caccggcgtg catgcgctgc tcggcctgca taacgacagt 960

ttctacggcc tgcgcgacgg tagcagtaaa accgctctgc tttatggtca tggtctgggc 1020

gcagaggtta aaggtatcgg atctgatggc gcacttcgtc cgggagccga cacatggcgc 1080

attgccagtt acggcaccac gccgctcagc gaaaactggt ctgttgcccc ggcaatgctg 1140

gcgcaacgca gtaaagaccg ctatgccgat ggcgacagct atcagtgggc aacattcaac 1200

ctgcgtctga ttcaggcaat caatcagaat ttcgctctcg cctacgaagg cagctaccag 1260

tacatggatc ttaaacccga aggttataac gatcgtcagg cggtgaacgg tagcttctac 1320

aagctcacct tcgccccgac atttaaggtc ggcagtatcg gtgatttctt cagtcgcccg 1380

gagattcgtt tctatacctc ctggatggac tggagcaaaa aactgaataa ttacgccagc 1440

gacgacgccc tgggcagtga cggttttaac tcgggcggcg aatggtcttt cggtgtgcag 1500

atggaaacct ggttctgacg cttacgcctg atgacaggaa tagccggggg tcagagcatc 1560

tttgtcaccc cggactcaac taagacgcag aaaaagcgct cccgtgaacg cgggacgaca 1620

acataaaaat gtttaagcct taagagggta ctatggattt tgaacagatt tcctgctcgc 1680

tgcttccgct tcttggaggc aaagaaaata tcgccagcgc cgcgcactgc gccacgcgcc 1740

tgcgcctggt gctggtcgat gattcgctgg ccgaccagca ggccatcggc aaagttgaag 1800

gggtgaaggg ctgttttcgt aatgccggac agatgcagat tattttcggc accggggtgg 1860

taaataaggt ctacgctgcc tttactcagg cggcgggtat tagcgaatcc agcaaatcgg 1920

aagccgccga catcgcggca aaaaagctca atccgttcca gcgcatcgcc cgcctgctat 1980

caaacatctt cgtgccgata atccctgcca tcgtcgcctc tggtctgctg atgggcctgc 2040

tgggaatggt caaaacatac ggctgggttg acccgggcaa cgccatctac atcatgctgg 2100

atatgtgcag ctcggcggca tttatcattc tgccgattct gattggcttt accgccgccc 2160

gcgaattcgg cggtaatcct tatctcggcg cgacgcttgg cggcattctg actcatccag 2220

cgctgactaa cgcctggggc gtggccgcgg gtttccacac catgaacttt ttcggcttcg 2280

aaattgccat gatcggctat cagggtacgg tgttcccggt actgctggca gtatggttta 2340

tgagcatcgt tgagaagcag ttgcgtcgcg caatccccga tgccctggat ttgatcctga 2400

cgccgttcct gacggtgatt atatccggtt ttatcgccct gttgattatc ggcccggccg 2460

gtcgcgcact gggcgacggt atctcgtttg tcctcagcac cctgattagc cacgccggct 2520

ggctcgccgg gttactgttt ggcggtctct attcagttat cgtcattacc ggtattcatc 2580

acagcttcca tgcggttgaa gccgggttgc tgggcaatcc ctccatcggc gtcaacttcc 2640

tgctgccgat ttgggcgatg gccaacgtcg ctcagggcgg agcctgtctg gcggtgtggt 2700

tcaaaaccaa agatgcaaaa attaaagcca ttactctgcc ctcggcgttt tccgccatgc 2760

tgggcatcac cgaggcggcg atttttggta ttaacctgcg ctttgtgaag ccatttattg 2820

cggcgctgat tggtggtgcg gcgggcggcg catgggtggt atctgtacac gtctacatga 2880

ccgcggtcgg cttgacagcg atccccggca tggccatcgt gcaggccagt tcgctgttga 2940

actacattat cgggatggtt atcgcctttg gcgtcgcctt tacggtctcc ctggttttga 3000

aatacaaaac ggacgctgaa taatgtctct tccatcacga ctgcctgcga ttttgcaggc 3060

cgtaatgcag ggccagccgc gcgcgctggc cgatagccac tatccgcgct ggcaccatgc 3120

gccggtcacc gggctgatga acgaccccaa cggctttatc gaatttgccg gacgctatca 3180

tctgttttat cagtggaacc cgctcgcctg cgatcatacg tttaagtgct gggcgcactg 3240

gagttccatc gatctgctgc actggcagca tgagcccatt gcgctgatgc cggacgaaga 3300

gtatgaccgt aacggctgct actccggcag cgcggtggat aacaacggta cgcttaccct 3360

gtgctatacc ggcaacgtga agtttgccga gggaggcgaa accgcctggc aatgcctggc 3420

aacggaaaac gctgacggca ccttccgcaa aatcggtccg gtcctgccgc tgccggaggg 3480

ctacaccggc cacgtgcgcg acccaaaagt ctggcgacac gaagacctgt ggtacatggt 3540

gctgggcgcg caggatcggc aaaagcgcgg caaggtgctg ctgttcagct ctgcggatct 3600

ccatcagtgg acgagtatgg gtgaaatcgc cggccacggc atcaatggcc tcgacgacgt 3660

cggctatatg tgggagtgcc cggatctttt tccactcggc gaccagcata ttctaatctg 3720

ctgtccgcag gggattgccc gtgaggaaga gtgctacctg aacacctacc cggcagtatg 3780

gatggcgggc gagtttgatt acgctgctgg cgctttcaga cacggcgaac tgcacgaact 3840

ggacgccggg tttgagttct acgccccgca aaccatgctt accagtgatg gccgtcgtct 3900

gctggtcggc tggatgggcg tgccggaggg cgaagagatg cttcagccga ccctgaacaa 3960

cggctggatc catcagatga cctgcctgcg tgagctggag tttatcaacg gtcagctcta 4020

tcagcgtccg ctacgggaac tgagcgccct gcgcggtgaa gcgaacggct ggtcggggaa 4080

cgccctgccg ctggcaccga tggaaatcga tttgcaaacc cgcgggggcg atatgttgag 4140

cctcgatttt ggcggcgtat taacccttga gtgcgatgcc agcggactcc gcctggcccg 4200

acgcagtctc gccagtgacg agatgcatta tcgttactgg cgcggaaacg tccgctcgct 4260

gcgtgttttc atcgaccagt cgagcgtgga gattttcata aacggcggtg aaggggtgat 4320

gagcagccgc tacttcccgg cctgctccgg tcagctaaca ttctccggca tcacgccgga 4380

cgcattctgc tactggccgc tgcgaacttg catggtagaa taa 4423

<210> 16
<211> 3883
<212> DNA
<213> Eastern equine encephalomyelitis virus

<400> 16
ctatattgct gaaggtacag gcgtttccat aactatttgc tcgcgttttt tactcaagaa 60
gaaaatgcca aatagcaaca tcaggcagac aatacccgaa attgcgaaga aaactgtctg 120
gtagcctgcg tggtcaaaga gtatcccagt cggcgttgaa agcagcacaa tcccaagcga 180
actggcaatt tgaaaaccaa tcagaaagat cgtcgacgac aggcgcttat caaagtttgc 240
cacgctgtat ttgaagacgg atatgacaca aagtggaacc tcaatggcat gtaacaactt 300
cactaatgaa ataatccagg ggttaacgaa cagcgcgcag gaaaggatac gcaacgccat 360
aatcacaact ccgataagta atgcattttt tggccctacc cgattcacaa agaaaggaat 420
aatcgccatg cacagcgctt cgagtaccac ctggaatgag ttgagataac catacaggcg 480
cgttcctaca tcgtgtgatt cgaataaacc tgaataaaag acaggaaaaa gttgttgatc 540
aaaaatgtta tagaaagacc acgtccccac aataaatatg acgaaaaccc agaagtttcg 600
atccttgaaa actgcgataa aatcctcttt ttttacccct cccgcatctg ccgctacgca 660
ctggtgatcc ttatctttaa aacgcatgtt gatcatcata aatacagcgc caaatagcga 720
gaccaaccag aagttgatat ggggactgat actaaaaaat atgccggcaa agaacgcgcc 780
aatagcatag ccaaaagatc cccaggcgcg cgctgttcca tattcgaaat gaaaatttcg 840
cgccattttt tcggtgaagc tatcaagcaa accgcatccc gccagatacc ccaagccaaa 900
aaatagcgcc cccagaatta gacctacaga aaaattgctt tgcagtaacg gttcataaac 960
gtaaatcata aacggtccgg tcaagaccag gatgaaactc atacaccaga tgagcggttt 1020
cttcagaccg agtttatcct gaacgatgcc gtagaacatc ataaatagaa tgctggtaaa 1080
ctggttgacc gaataaagtg tacctaattc cgtccctgtc aaccctagat gtcctttcag 1140
ccaaatagcg tataacgacc accacagcga ccaggaaata aaaaagagaa atgagtaact 1200
ggatgcaaaa cgatagtacg catttctgaa tggaatattc agtgccataa ttacctgcct 1260
gtcgttaaaa aattcacgtc ctatttagag ataagagcga cttcgccgtt tacttctcac 1320
tattccagtt cttgtcgaca tggcagcgct gtcattgccc ctttcgccgt tactgcaagc 1380
gctccgcaac gttgagcgag atcgataatt cgtcgcattt ctctctcatc tgtagataat 1440
cccgtagagg acagacctgt gagtaacccg gcaacgaacg catctcccgc ccccgtgcta 1500
tcgacacaat tcacagacat tccagcaaaa tggtgaactt gtcctcgata acagaccacc 1560
accccttctg cacctttagt caccaacagc atggcgatct catactcttt tgccagggcg 1620
catatatcct gatcgttctg tgtttttcca ctgataagtc gccattcttc ttccgagagc 1680
ttgacgacat ccgccagttg tagcgcctgc cgcaaacaca agcggagcaa atgctcgtct 1740
tgccatagat cttcacgaat attaggatcg aagctgacaa aacctccggc atgccggatc 1800
gccgtcatcg cagtaaatgc gctggtacgc gaaggctcgg cagacaacgc aattgaacag 1860

agatgtaacc attcgccatg tcgccagcag ggcaagtctg tcgtctctaa aaaaagatcg 1920

gcactggggc ggaccataaa cgtaaatgaa cgttcccctt gatcgttcag atcgacaagc 1980

accgtggatg tccggtgcca ttcatcttgc ttcagatacg tgatatcgac tccctcagtt 2040

agcagcgttc tttgcattaa cgcaccaaaa ggatcatccc ccacccgacc tataaaccca 2100

cttgttccgc ctaatctggc gattcccacc gcaacgttag ctggcgcgcc gccaggacaa 2160

ggcagtaggc gcccgtctga ttctggcaag agatctacga ccgcatcccc taaaacccat 2220

actttggctg acattttttt cccttaaatt catctgagtt acgcatagtg ataaacctct 2280

ttttcgcaaa atcgtcatgg atttactaaa acatgcatat tcgatcacaa aacgtcatag 2340

ttaacgttaa catttgtgat attcatcgca tttatgaaag taagggactt tatttttata 2400

aaagttaacg ttaacaattc accaaatttg cttaaccagg atgattaaaa tgacgcaatc 2460

tcgattgcat gcggcgcaaa acgccctagc aaaacttcat gagcaccggg gtaacacttt 2520

ctatccccat tttcacctcg cgcctcctgc cgggtggatg aacgatccaa acggcctgat 2580

ctggtttaac gatcgttatc acgcgtttta tcaacatcat ccgatgagcg aacactgggg 2640

gccaatgcac tggggacatg ccaccagcga cgatatgatc cactggcagc atgagcctat 2700

tgcgctagcg ccaggagacg ataatgacaa agacgggtgt ttttcaggta gtgctgtcga 2760

tgacaatggt gtcctctcac ttatctacac cggacacgtc tggctcgatg gtgcaggtaa 2820

tgacgatgca attcgcgaag tacaatgtct ggctaccagt cgggatggta ttcatttcga 2880

gaaacagggt gtgatcctca ctccaccaga aggaatcatg cacttccgcg atcctaaagt 2940

gtggcgtgaa gccgacacat ggtggatggt agtcggggcg aaagatccag gcaacacggg 3000

gcagatcctg ctttatcgcg gcagttcgtt gcgtgaatgg accttcgatc gcgtactggc 3060

ccacgctgat gcgggtgaaa gctatatgtg ggaatgtccg gactttttca gccttggcga 3120

tcagcattat ctgatgtttt ccccgcaggg aatgaatgcc gagggataca gttaccgaaa 3180

tcgctttcaa agtggcgtaa tacccggaat gtggtcgcca ggacgacttt ttgcacaatc 3240

cgggcatttt actgaacttg ataacgggca tgacttttat gcaccacaaa gctttttagc 3300

gaaggatggt cggcgtattg ttatcggctg gatggatatg tgggaatcgc caatgccctc 3360

aaaacgtgaa ggatgggcag gctgcatgac gctggcgcgc gagctatcag agagcaatgg 3420

caaacttcta caacgcccgg tacacgaagc tgagtcgtta cgccagcagc atcaatctgt 3480

ctctccccgc acaatcagca ataaatatgt tttgcaggaa aacgcgcaag cagttgagat 3540

tcagttgcag tgggcgctga agaacagtga tgccgaacat tacggattac agctcggcac 3600

tggaatgcgg ctgtatattg ataaccaatc tgagcgactt gttttgtggc ggtattaccc 3660

acacgagaat ttagacggct accgtagtat tcccctcccg cagcgtgaca cgctcgccct 3720

aaggatattt atcgatacat catccgtgga agtatttatt aacgacgggg aagcggtgat 3780

gagtagtcga atctatccgc agccagaaga acgggaactg tcgctttatg cctcccacgg 3840

agtggctgtg ctgcaacatg gagcactctg gctactgggt taa 3883

## Description

The present invention relates to the fermentative production of a saccharide of interest. Disclosed are microbial cells being capable to the saccharide of interest, wherein said microbial cells utilizes a mixed monosaccharide feedstock as main carbon- and energy source during fermentation. Also disclosed are methods for producing the saccharide of interest by utilizing said microbial cells.

### Background

Human milk comprises a complex mixture of carbohydrates, fats, proteins, vitamins, minerals and trace elements. The most predominant fraction of human milk consists of carbohydrates. The carbohydrate fraction within human milk can be further divided into (i) lactose and (ii) oligosaccharides (human milk oligosaccharides, HMOs). Whereas the disaccharide lactose (galactose-β1,4-glucose) is used as an energy source by the baby, the oligosaccharides are not metabolized by the infant.

The fraction of oligosaccharides accounts for up to one tenth of the total carbohydrate fraction and presumably consists of more than 150 structurally distinct oligosaccharides. The occurrence and concentration of these complex oligosaccharides are specific to humans and thus cannot be found in the milk of other mammals including dairy farm animals in large quantities.

The most prominent human milk oligosaccharides are 2'-fucosyllactose (2'-FL) and 3-fucosyllactose (3-FL) which together can constitute up to 1/3 of the total HMO fraction. Further prominent HMOs are lacto-*N*-tetraose (LNT), lacto-*N*-neotetraose (LNnT) and lacto-*N*-fucopentaose I (LNFP-I). Besides these neutral oligosaccharides, also acidic HMOs can be found in human milk such as 3'-sialyllactose (3'-SL), 6'-sialyllactose (6'-SL), 3-fucosyl-3'-sialyllactose, sialyl-lacto-*N*-tetraose, and disialyl-lacto-*N*-tetraose.

Notably, the vast majority of HMOs comprise a galactose-β1,4-glucose moiety at their reducing end, which gets extended by the addition of monosaccharide moieties such as *N*-acetylglucosamine (GlcNAc) and/or fucose and/or galactose and/or *N*-acetylneuraminic acid (NeuNAc). The structures of HMOs are closely related to epitopes of epithelial cell surface glycoconjugates, the Lewis histoblood group antigens such as Lewis x (LeX). The structural similarity of HMOs to epithelial epitopes accounts for the protective properties of HMOs against bacterial pathogens.

The presence of oligosaccharides in human milk is known for a long time and the physiological functions of these oligosaccharides were subject to medical research for many decades. For some of the more abundant human milk oligosaccharides, specific functions have already been identified.

Besides causing local effects in the intestinal tract as indicated herein before, HMOs have also been shown to elicit systemic effects in infants by entering their systemic circulation. Also, the impact of HMOs on protein-carbohydrate interactions, e.g. selectin-leukocyte binding, can modulate immune responses and reduce inflammatory responses. In addition, it becomes more and more recognized that HMOs represent key substrates for the development of infants' microbiomes.

Due to the well-studied beneficial properties of various carbohydrates in general, and in particular of prebiotic oligosaccharides, but because of their limited availability from natural sources, an efficient and cost-effective production process for monosaccharides (e.g. of L-fucose, *N*-acetylneuraminic acid), disaccharides (e.g. lacto-*N*-biose) and oligosaccharides (e.g. 2'-FL, LNnT) is highly desirable.

Attempting for large scale production of functional carbohydrates, chemical routes to some of these carbohydrates were developed. However, such methods involve the use of several noxious chemicals, which impose the risk to contaminate the final product. At least large-scale quantities as well as qualities of functional carbohydrates being sufficient for their use in food applications cannot be provided until today through chemical synthesis.

To bypass the drawbacks that are associated with the chemical synthesis of human milk oligosaccharides, several enzymatic methods and fermentative approaches for their production were developed. Fermentative production processes have been developed for several carbohydrates such as L-fucose, N-acetylneuraminic acid,
2'-fucosyllactose, 3-fucosyllactose, lacto-*N*-tetraose, lacto-*N*-neotetraose, 3'-sialyllactose and 6'-sialyllactose. These production processes typically use genetically engineered bacterial cells such as recombinant *Escherichia coli.*

Usually, fermentative production processes as well as biocatalytic reactions to produce HMOs are based on exogenously added lactose as initial acceptor substrate for the HMO to be produced. One or more monosaccharides are added to lactose in these processes (US 7,521,212 B1; Albermann et al., (2001) Carbohydr. Res. 334 (2) p. 97-103). The addition of monosaccharides to lactose can either be catalyzed by glycosyltransferases or by glycosidases using suitable activated monosaccharide substrates. In addition, additional monosaccharides can be added to lactose by transglycosidase reactions.

In particular the fermentative production of HMOs proved to be efficient, because the nucleotide-activated monosaccharides that are required but difficult to synthesize are provided by the metabolism of the microbial cells that are employed. The biosynthesis pathway for nucleotide-activated monosaccharides usually originates from the primary metabolism of the host cell on the level of glucose-6-phosphate or fructose-6-phoshate. The biosynthesis pathway for UDP-galactose (UDP-Gal) originates from glucose-6-phosphate while the biosynthesis of GDP-fucose, UDP-N-acetylglucosamine and CMP-*N*-acetylneuraminic acid originates from fructose-6-phosphate.

The efficient biosynthesis of nucleotide-activated monosaccharides in a microbial host cell, and consequently the production of desired carbohydrates by fermentative processes, clearly rely on the continuous supply of sugar phosphates such as glucose-6-phosphate and/or fructose-6-phosphate.

A major problem in said fermentative processes, usually based on the consumption of a simple and inexpensive carbon- and energy source (e.g. glycerol, glucose, sucrose) by the microbial host cell, is the limited availability of such phosphorylated/ activated saccharides in said host cell (e.g. due to competing reactions), vastly impairing the carbon flux of the product biosynthesis pathway, thus, the productivity of these processes.

To overcome aforementioned drawbacks, improved means and methods for the production of HMOs were developed. For example, WO 2012/007481 A2 discloses a engineered organism being capable to produce a saccharide, an activated saccharide, a nucleoside, a glycoside, a glycolipid and a glycoprotein, wherein said engineered organism expresses i) a gene encoding for a carbohydrate hydrolase in combination with a gene encoding for a carbohydrate kinase, ii) a gene encoding for a carbohydrate synthase, or iii) a gene encoding for a carbohydrate phosphorylase, so that said organism is capable to split a disaccharide, oligosaccharide, polysaccharide or a mixture thereof into an activated saccharide and a saccharide and wherein said organism is further genetically modified such that at least one other gene than any of the introduced genes of said organism is rendered less-functional or non-functional, and wherein said other gene encodes for an enzyme which converts said activated saccharide into biomass and/or biocatalytic enzymes. Said engineered organism is capable of generating desired carbohydrates while utilizing a disaccharide such as sucrose, an oligosaccharide, a polysaccharide or a mixture thereof.

However, producing a desired compound by said engineered organism using sucrose as sole carbon and energy source has a major drawback in that it is difficult to sterilize sucrose. The most desirable method for sterilization is heat sterilization. However, heat sterilization of sucrose results in a considerable degree of hydrolyzation counteracting the applicability of the method described in WO 2012/007481 A2.

As an alternative to heat sterilization, sterile filtration of a sucrose solution can be employed, but sterile filtration bears a high risk of contamination leading to growth of undesired bacterial cells, in particular in industrial-scale fermentation.

Nevertheless, sucrose represents the most attractive carbon source for biotechnological applications due to its availability and low costs.

Hence, it is desired to provide microbial cells for the fermentative production of a desired carbohydrate that is capable of producing the desired carbohydrate when cultivated in the presence of an inexpensive feedstock as main carbon and energy source wherein said feedstock does not have to be hydrolyzed by the microbial cell to be utilizable by the cell's metabolism, and a method for producing a carbohydrate of interest by means of fermentation by cultivating said microbial cell in the presence of said feedstock as main carbon and energy source.

The object is solved in that genetically engineered microbial cells are provided which are capable of producing a desired carbohydrate when cultivated on a mixed monosaccharide feedstock as main carbon and energy source, wherein said mixed monosaccharide feedstock consists of glucose and at least one additional monosaccharide selected from the group consisting of fructose and galactose, and wherein the microbial cells possess an increased intracellular availability of at least one sugar phosphate, and by providing a method for the fermentative production of a carbohydrate of interest which method comprises cultivating said genetically engineered microbial cells in the presence of said mixed monosaccharide feedstock as main carbon and energy source.

### Summary

According to a first aspect, provided is a genetically engineered microbial cell for the production of a carbohydrate of interest, wherein said microbial cell possesses an increased intracellular availability of at least one sugar phosphate, and is capable of producing a carbohydrate of interest when cultivated in the presence of a mixed monosaccharide feedstock as main carbon and energy source, wherein said mixed monosaccharide feedstock consists of glucose and at least one additional monosaccharide selected from the group consisting of fructose and galactose.

According to a second aspect, provided is the use of a genetically engineered microbial cell as described herein for the production of a carbohydrate of interest, wherein said microbial cell is cultivated in the presence of a monosaccharide mixture consisting of glucose and at least one additional monosaccharide from the group of fructose and galactose.

According to a third aspect, provided is a method for the fermentative production of a carbohydrate of interest, the method comprising the steps of:
a) providing a genetically engineered microbial cell that is capable of producing the carbohydrate of interest, wherein said microbial cell possesses an increased intracellular availability of at least one sugar phosphate;
b) cultivating said genetically engineered microbial cell in a culture medium that is permissive for the production of said carbohydrate of interest, wherein the culture medium comprises a mixed monosaccharide feedstock as main carbon and energy source, wherein said mixed monosaccharide feedstock consist of glucose and at least one additional monosaccharide selected from the group consisting of fructose and galactose; and
c) recovering said carbohydrate of interest.

In a fourth aspect, provided is the use of the carbohydrate of interest that has been produced by the genetically engineered microbial cell and/or by the method described herein for the manufacturing a pharmaceutical and/or nutritional composition.

### Brief description of the drawings

- Fig. 1: shows a schematic representation of an exemplary wild-type microbial cell (e.g. *E. coli)* illustrating naturally occurring metabolic pathways leading to and originating from the sugar phosphates fructose-1-phosphate and glucose-6-phosphate.
- Fig. 2: shows a schematic representation of an exemplary genetically modified microbial cell of the invention, wherein genetic modifications, leading to an increased intracellular glucose-6-phosphate availability, are indicated.
- Fig. 3: shows a schematic drawing of another exemplary embodiment of a genetically engineered microbial cell of the invention, wherein genetic modifications, leading to an increased intracellular glucose-6-phosphate availability, are indicated.
- Fig. 4: shows a schematic drawing of another exemplary embodiment of a genetically engineered microbial cell of the invention, wherein genetic modifications, leading to an increased intracellular glucose-6-phosphate availability, are indicated.
- Fig. 5: shows a schematic drawing of another exemplary embodiment of a genetically engineered microbial cell of the invention, wherein genetic modifications, leading to an increased intracellular glucose-6-phosphate availability and an increased intracellular fructose-6-posphate availability, are indicated.
- Fig. 6: shows a schematic drawing of another exemplary embodiment of a genetically engineered microbial cell of the invention, wherein genetic modifications, leading to an increased intracellular glucose-6-phosphate availability and an increased intracellular fructose-6-posphate availability, are indicated.
- Fig. 7: shows a schematic drawing of another exemplary embodiment of a genetically engineered microbial cell of the invention, wherein genetic modifications, leading to an increased intracellular glucose-6-phosphate availability and an increased intracellular fructose-6-posphate availability, are indicated.
- Fig. 8: shows a schematic drawing of another exemplary embodiment of a genetically engineered microbial cell of the invention, wherein genetic modifications, leading to an increased intracellular glucose-6-phosphate availability and an increased intracellular fructose-6-posphate availability, are indicated.
- Fig. 9: shows a schematic drawing of another exemplary embodiment of a genetically engineered microbial cell of the invention, wherein genetic modifications, leading to an increased intracellular glucose-6-phosphate availability and an increased intracellular fructose-6-posphate availability, are indicated.
- Fig. 10: shows a schematic drawing of another exemplary embodiment of a genetically engineered microbial cell of the invention, wherein genetic modifications, leading to an increased intracellular availability of glucose, glucose-6-phosphate and fructose-6-posphate, are indicated.
- Fig. 11: shows a schematic drawing of another exemplary embodiment of a genetically engineered microbial cell of the invention, wherein genetic modifications, leading to an increased intracellular availability of mannose and/or mannose-6-phosphate, are indicated.
- Fig. 12: shows a schematic drawing of another exemplary embodiment of a genetically engineered microbial cell of the invention, wherein genetic modifications, leading to an increased intracellular availability of galactose-1-phosphate and fructose-6-phosphate, are indicated.

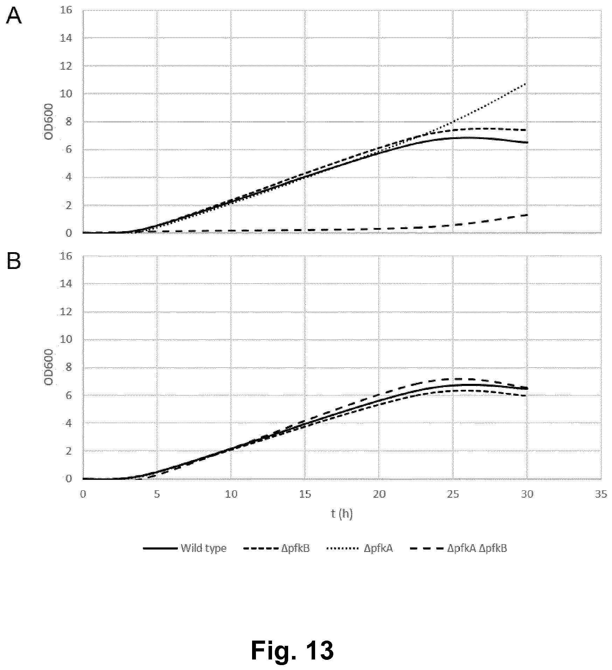

- Fig. 13: displays growth characteristics of *E. coli* strains during cultivation on glucose (A) or on a mixed-monosaccharide feedstock consisting of glucose and fructose (B) as sole carbon- and energy source.

### Detailed description

According to the first aspect, provided is a genetically engineered microbial cell that is capable to produce a carbohydrate of interest. In an additional embodiment, the carbohydrate of interest is a carbohydrate that does not naturally occur in the wild type progenitor of the genetically engineered microbial cell.

The microbial cell possesses an increased intracellular availability of at least one sugar phosphate as compared to the intracellular availability of said at least one sugar phosphate in the corresponding wild-type cell. The genetically engineered microbial cell is capable to produce said carbohydrate of interest, and produces said carbohydrate of interest when being cultivated in a culture medium comprising a mixed monosaccharide feedstock as main carbon and energy source for the microbial cell, wherein the mixed monosaccharide feedstock consists of glucose and at least one additional monosaccharide selected from the group consisting of fructose and galactose.

The genetically engineered microbial cell is able to produce the carbohydrate of interest due to being genetically engineered. Hence, the genetically engineered microbial cell expresses one or more heterologous gene(s), wherein the activity of the polypeptide(s) encoded by said heterologous gene(s) enable(s) the microbial cell to synthesize the carbohydrate of interest.

The term "functional gene" as used herein, refers to a nucleic acid molecule comprising a nucleotide sequence which encodes a protein or polypeptide, and which also contains regulatory sequences operably linked to said protein-coding nucleotide sequence such that the nucleotide sequence which encodes the protein or polypeptide can be expressed in/by the microbial cell bearing said functional gene. Thus, when cultivated at conditions that are permissive for the expression of the functional gene, said functional gene is expressed, and the microbial cell expressing said functional gene typically comprises the protein or polypeptide that is encoded by the protein coding region of the functional gene. As used herein, the terms "nucleic acid" and "polynucleotide" refer to a deoxyribonucleotide or ribonucleotide polymer in either single- or double-stranded form, and unless otherwise limited, encompasses known analogues of natural nucleotides that hybridize to nucleic acids in a manner similar to naturally occurring nucleotides. Unless otherwise indicated, a particular nucleic acid sequence includes the complementary sequence thereof.

The term "operably linked" as used herein, shall mean a functional linkage between a nucleic acid expression control sequence (such as a promoter, signal sequence, or array of transcription factor binding sites) and a second nucleic acid sequence, wherein the expression control sequence affects transcription and/or translation of the nucleic acid corresponding to the second sequence. Accordingly, the term "Promoter" designates DNA sequences which usually "precede" a gene in a DNA polymer and provide a site for initiation of the transcription into mRNA. "Regulator" DNA sequences, also usually "upstream" of (i.e., preceding) a gene in a given DNA polymer, bind proteins that determine the frequency (or rate) of transcriptional initiation. Collectively referred to as "promoter/regulator" or "control" DNA sequence, these sequences which precede a selected gene (or series of genes) in a functional DNA polymer cooperate to determine whether the transcription (and eventual expression) of a gene will occur. DNA sequences which "follow" a gene in a DNA polymer and provide a signal for termination of the transcription into mRNA are referred to as transcription "terminator" sequences.

The term "recombinant", as used herein with reference to a bacterial host cell indicates that the bacterial cell replicates a heterologous nucleic acid, or expresses a peptide or protein encoded by a heterologous nucleic acid (i.e., a sequence that is "foreign to said cell"). Recombinant cells can contain genes that are not found within the native (non-recombinant) form of the cell. Recombinant cells can also contain genes found in the native form of the cell wherein the genes are modified and re-introduced into the cell by artificial means. The term also encompasses cells that contain a nucleic acid endogenous to the cell that has been modified without removing the nucleic acid from the cell; such modifications include those obtained by gene replacement, site-specific mutation, and related techniques. Accordingly, a "recombinant polypeptide" is one which has been produced by a recombinant cell. A "heterologous sequence" or a "heterologous nucleic acid", as used herein, is one that originates from a source foreign to the particular host cell (e.g. from a different species), or, if from the same source, is modified from its original form. Thus, a heterologous nucleic acid operably linked to a promoter is from a source different from that from which the promoter was derived, or, if from the same source, is modified from its original form. The heterologous sequence may be stably introduced, e.g. by transfection, transformation, conjugation or transduction, into the genome of the host microorganism cell, wherein techniques may be applied which will depend on the host cell the sequence is to be introduced. Various techniques are known to a person skilled in the art and are, e.g., disclosed in Sambrook et al., Molecular Cloning: A Laboratory Manual, 2nd Ed., Cold Spring Harbor Laboratory Press, Cold Spring Harbor, N.Y. (1989).

Accordingly, a "genetically engineered host cell" or "genetically engineered microbial cell" is understood as a bacterial cell or a yeast cell which has been transformed or transfected, or is capable of transformation or transfection by an exogenous polynucleotide sequence.

Thus, the nucleic acid sequences as used in the present invention, may, e.g., be comprised in a vector which is to be stably transformed/transfected or otherwise introduced into host microorganism cells.

A great variety of expression systems can be used to produce the polypeptides of the invention. Such vectors include, among others, chromosomal, episomal and virus-derived vectors, e.g., vectors derived from bacterial plasmids, from bacteriophage, from transposons, from yeast episomes, from insertion elements, from yeast chromosomal elements, from viruses, and vectors derived from combinations thereof, such as those derived from plasmid and bacteriophage genetic elements, such as cosmids and phagemids. The expression system constructs may contain control regions that regulate as well as engender expression. Generally, any system or vector suitable to maintain, propagate or express polynucleotides and to synthesize a polypeptide in a host may be used for expression in this regard. The appropriate DNA sequence may be inserted into the expression system by any of a variety of well-known and routine techniques, such as, for example, those set forth in Sambrook *et al*., *supra*.

The art is rich in patent and literature publications relating to "recombinant DNA" methodologies for the isolation, synthesis, purification and amplification of genetic materials for use in the transformation of selected host organisms. Thus, it is common knowledge to transform host organisms with "hybrid" viral or circular plasmid DNA which includes selected exogenous (i.e. foreign or "heterologous") DNA sequences. The procedures known in the art first involve generation of a transformation vector by enzymatically cleaving circular viral or plasmid DNA to form linear DNA strands. Selected foreign DNA strands usually including sequences coding for desired protein product are prepared in linear form through use of the same/similar enzymes. The linear viral or plasmid DNA is incubated with the foreign DNA in the presence of ligating enzymes capable of effecting a restoration process and "hybrid" vectors are formed which include the selected exogenous DNA segment "spliced" into the viral or circular DNA plasmid.

The term "nucleotide sequence encoding..." generally refers to any polyribonucleotide or polydeoxyribonucleotide, which may be unmodified RNA or DNA or modified RNA or DNA, and generally represents the portion of a gene which encodes a certain polypeptide or protein. The term includes, without limitation, single- and double-stranded DNA, DNA that is a mixture of single- and double-stranded regions or single-, double- and triple-stranded regions, single- and double-stranded RNA, and RNA that is mixture of single- and double-stranded regions, hybrid molecules comprising DNA and RNA that may be single-stranded or, more typically, double-stranded, or triple-stranded regions, or a mixture of single- and double-stranded regions. The term also encompasses polynucleotides that include a single continuous region or discontinuous regions encoding the polypeptide (for example, interrupted by integrated phage or an insertion sequence or editing) together with additional regions that also may contain coding and/or non-coding sequences.

The term "variant(s)" as used herein, refers to a polynucleotide or polypeptide that differs from a reference polynucleotide or polypeptide respectively, but retains the essential (enzymatic) properties of the reference polynucleotide or polypeptide. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Nonnaturally occurring variants of polynucleotides and polypeptides may be made by mutagenesis techniques, by direct synthesis, and by other recombinant methods known to the persons skilled in the art.

Within the scope of the present invention, also nucleic acid/polynucleotide and polypeptide polymorphic variants, alleles, mutants, and interspecies homologs are comprised by those terms, that have an amino acid sequence that has greater than about 60% amino acid sequence identity, 65%, 70%, 75%, 80%, 85%, 90%, preferably 91%, 92%, 93%, 94%, 95%, 96%, 97%, 98% or 99% or greater amino acid sequence identity, preferably over a region of at least about 25, 50, 100, 200,500, 1000, or more amino acids, to a polypeptide encoded by a wildtype protein.

Accordingly, a "functional variant" of any of the genes/proteins disclosed therein, is meant to designate sequence variants of the genes/proteins still retaining the same or somewhat lesser activity of the gene or protein the respective fragment is derived from.

The genetically engineered microbial cell possesses at least one monosaccharide transporter for translocating at least one monosaccharide from the culture medium said microbial cell is cultivated in into its cytoplasm. Said at least one monosaccharide transporter translocates a monosaccharide selected from the group consisting of glucose, fructose and galactose.

The monosaccharide that has been translocated across the cell membrane has to be phosphorylated in order to become accessible for the cell's metabolism. Depending on the monosaccharide transporter that has translocated the monosaccharide across the cell membrane, the monosaccharide(s) are either directly phosphorylated, i.e. while being transferred into the cell, or are subsequently phosphorylated by a suitable kinase. Translocation of the monosaccharide by a phosphoenolpyruvate:sugar phosphotransferase dependent transport (PEP-PTS) leads to direct phosphorylation, whereas translocation of a monosaccharide by means of a phosphoenolpyruvate:sugar phosphotransferase independent transport (non-PEP-PTS) requires subsequent phosphorylation of the monosaccharide by an intracellular kinase.

In an additional and/or alternative embodiment, the microbial cell comprises a glucose-translocating phosphotransferase system (PtsG). The glucose-translocating phosphotransferase system catalyzes the phosphorylation of incoming glucose concomitantly with its translocation across the cell membrane.

The general mechanism of the Pts system is the following: a phosphoryl group from phosphoenolpyruvate (PEP) is transferred via a signal transduction pathway, to enzyme I (EI) which in turn transfers it to a phosphoryl carrier, the histidine protein (HPr). Phospho-HPr then transfers the phosphoryl group to a sugar-specific permease, a membrane-bound complex known as enzyme 2 (Ell), which transports the sugar to the cell. Ell consists of at least three structurally distinct domains IIA, IIB and IIC. These can either be fused together in a single polypeptide chain or exist as two or three interactive chains, formerly called enzymes II (EII) and III (EIII).

The first domain (IIA or EIIA) carries the first permease-specific phosphorylation site, a histidine which is phosphorylated by phospho-HPr. The second domain (IIB or EIIB) is phosphorylated by phospho-IIA on a cysteinyl or histidyl residue, depending on the sugar transported. Finally, the phosphoryl group is transferred from the IIB domain to the sugar substrate concomitantly with the sugar uptake processed by the IIC domain. This third domain (IIC or EIIC) forms the translocation channel and the specific substrate-binding site.

Thus, the PtsG system acquires exogenous glucose and provides glucose-6-phosphate in the microbial cell. Glucose-6-phosphate can be utilized in the UDP-galactose biosynthesis pathway and/or converted to fructose-6-phosphate which in turn may be used for generating energy-rich triphosphates in the central metabolism and/or, for example, in the biosynthesis of nucleotide activated saccharides such as GDP-fucose.

In an additional and/or alternative embodiment, the genetically engineered microbial cell comprises a fructose transporter for translocating fructose (Frc) from the culture medium into the microbial cell's cytoplasm. A suitable fructose transporter for uptake of free fructose is an isoform (PtsG-F) as described by Kornberg et al. PNAS 97: 1808-1812 (2000)).

The internalized fructose may then be phosphorylated by a fructokinase (FrK) to provide fructose-6-phosphate (Fru-6-P). Fructose-6-phosphate may be utilized in the UDP-galactose biosynthesis pathway and/or in other metabolic pathways such as generating energy-rich triphosphates in the central metabolism and/or, for example, in the biosynthesis of nucleotide activated saccharides such as GDP-fucose.

In an additional and/or alternative embodiment, the genetically engineered microbial cell comprises a fructose-translocating phosphotransferase system (PtsF). The fructose-translocating phosphotransferase system catalyzes the phosphorylation of incoming fructose concomitantly with its translocation across the cell membrane.

Thus, the PtsF system acquires exogenous fructose and provides fructose-1-phosphate in the microbial cell. The PtsF system comprises a membrane-spanning protein FruA, a 1-phosphofructose kinase (FruK) and a diphosphoryl transfer protein FruB. Fructose is translocated by means of FruA and FruB to provide fructose-1-phosphate in the cytoplasm. Fructose-1-phosphate can be further phosphorylated by a phosphofructokinase (FruK) to yield fructose-1,6-bisphosphate which in turn may be used by the microbial cell for generating energy-rich triphosphates in the central metabolism.

Another suitable PtsF System comprises LevD, LevE, LevF and LevG. LevD is the fructose-specific phosphotransferase enzyme IIA component. LevE is the fructose-specific phosphotransferase enzyme IIB component. LevF is the fructose permease IIC component, LevG is the fructose permease IID component. Corresponding genes *levD*, *levE*, *levF* and *levG* are - for example known form *Bacillus subtilis* (strain 168). Said PtsF system provides fructose-1-phosphate in the cell.

In an additional and/or alternative embodiment, the genetically engineered microbial cell possesses an UDP-galactose biosynthesis pathway for intracellular formation of UDP-galactose (UDP-Gal). UDP-galactose is required as a substrate for galactosyltransferases wherein the activity of said galactosyltransferases may lead to the formation of galactosylated disaccharides or galactosylated oligosaccharides.

UDP-galactose may be provided by the microbial cells' naturally occurring metabolism, i.e. by the activity of a phosphoglucomutase catalyzing the interconversion of glucose1-phosphate and glucose 6-phosphate, an UTP-glucose-1-phosphate-uridyltransferase which catalyzes the formation of UDP-glucose from glucose-1-phosphate and UTP, and an UDP-glucose-4-epimerase catalyzing the reversible conversion of UDP-glucose to UDP-galactose.

The intracellular supply of UDP-galactose can be improved by genetic engineering in that one or more of the genes encoding the phosphoglucomutase, the UDP-glucose-1-phosphate-uridyltransferase, the UDP-glucose-4-epimerase, and functional variants thereof are overexpressed and/or in that one or more additional copies of one or more of the genes encoding one or more of the genes encoding the phosphoglucomutase, the UDP-glucose-1-phosphate-uridyltransferase, the UDP-glucose-4-epimerase, and functional variants thereof are expressed in the microbial cell. An example of a gene encoding a phosphoglucomutase is the *pgm* gene (acc. no. NP_415214) found in *E. coli* K-12, an example of a gene encoding an UDP-glucose-1-phosphate-uridyltransferase is the *E*. *coli galU* gene (acc. no. NP_415752) found in *E*. *coli* K-12, and an example of a gene encoding a UDP-glucose-4-epimerase is the *E*. *coli galE* gene (NP_415280) found in *E*. *coli K-12.*

The term "overexpression" or "overexpressed" as used herein refers to a level of enzyme or polypeptide expression that is greater than what is measured in a wild-type progenitor cell, i.e. a cell of the same species as the genetically engineered microbial cell and which cell that has not been genetically engineered.

Additionally and/or alternatively, intracellular UDP-galactose supply can be achieved or improved by feeding galactose to the microbial cells via the culture medium said microbial cell is cultivated in. The exogenously supplied galactose is taken up by the microbial cell and subsequently phosphorylated to galactose-1-phosphate which is then converted to UDP-galactose. In this GDP-galactose biosynthesis pathway, the genes encoding the enzymes possessing the required enzymatic activities are known in the literature (Groissoird et al., "Characterization, Expression, and Mutation of the Lactococcus lactis galPMKTE Genes, Involved in Galactose Utilization via the Leloir Pathway (2003) J. Bacteriol. 185(3) 870-878). The *galP* gene (acc. no. NP_417418) encodes a galactose-proton symporter. The *galM* gene (acc. no. NP_415277) encodes an aldolase 1-epimerase, the *galK* gene (acc. no. NP_415278) a galactokinase, the *galT* gene (acc. no. NP_415279) a galactose-1-phosphate uridyltransferase, and the *galE gene* an UDP-galactose-4-epimerase.

Hence, UDP-galactose biosynthesis can also be supplied by expression of genes encoding a galactose-proton symporter, a galactose kinase, and a galactose-1-phosphate uridyltransferase in the microbial cell, or improved by overexpression of the genes encoding a galactose-proton symporter, a galactose kinase, and a galactose-1-phosphate uridyltransferase in the microbial cell.

In another embodiment, the genetically engineered microbial cell possesses an GDP-fucose biosynthesis pathway for intracellular formation of GDP-L-fucose (GDP-Fuc). GDP-Fuc is a substrate of fucosyltransferases, and the enzymatic activity of fucosyltransferases may lead to the formation of fucosylated disaccharides or fucosylated oligosaccharides.

In an additional and/or alternative embodiment, the genetically engineered microbial cell possesses a GDP-L-fucose biosynthesis pathway comprising a mannose-6-phosphate isomerase, a phosphomannomutase, a mannose-1-phosphate-guanylyltransferase, a GDP-mannose-4,6-dehydratase and a GDP-L-fucose synthase.

The intracellular supply of GDP-L-fucose can be improved by genetic engineering in that one or more genes encoding a mannose-6-phosphate isomerase, a phosphomannomutase, a mannose-1-phosphate-guanylyltransferase, a GDP-mannose-4,6-dehydratase or a GDP-L-fucose synthase are overexpressed and/or additional copies of one or more of the genes encoding a mannose-6-phosphate isomerase, a phosphomannomutase, a mannose-1-phosphate-guanylyltransferase, a GDP-mannose-4,6-dehydratase and a GDP-L-fucose synthase, or functional variants thereof are expressed in the microbial cell. An example of a gene encoding a mannose-6-phosphate isomerase is the *E. coli manA* gene (acc. no. NP_416130) found in *E. coli* K-12, an example for a gene encoding a phosphomannomutase is the *E*. *coli manB* gene (acc. no. NP_416552) found in *E*. *coli* K-12, an example for a gene encoding a mannose-1-phosphate-guanylyltransferase is the *E*. *coli manC* gene (acc. no. NP_416553) found in *E*. *coli* K-12, an example for a gene encoding a GDP-mannose-4,6-dehydratase is the *E*. *coli gmd* gene (acc. no. NP_416557) found in *E*. *coli* K-12, and an example of a gene encoding a GDP-L-fucose synthase is the *E*. *coli wcaG* gene (acc. no. NP_416556) found in *E*. *coli* K-12.

Additionally and/or alternatively, GDP-L-fucose supply can be achieved or improved by feeding L-fucose to the microbial cells via the culture medium said microbial cell is cultivated in. The exogenously supplied L-fucose is taken up by the microbial cell and first phosphorylated to fucose-1-phosphate by the enzyme fucose kinase. Fucose-1-phosphate is subsequently converted to GDP-L-fucose by the enzymatic activity of the enzyme fucose-1-phosphate guanylyltransferase. Genes encoding the enzymes possessing the required enzymatic activities are known to the skilled artisan. Exemplarily, the *fkp* gene (acc. no. WP_010993080), encoding the bifunctional L-fucokinase/L-fucose 1-phosphat guanylyltranferase of *Bacteroides fragilis*, may be overexpressed within the genetically engineered host cell.

In another embodiment, the genetically engineered microbial cell possesses an UDP-*N*-acetylglucosamine biosynthesis pathway for intracellular formation of UDP-*N*-acetylglucosamine (UDP-GlcNAc), required for *N*-acetylglucosaminyltransferase reactions leading e.g. to the formation of *N*-acetylglucosaminylated di- or oligosaccharides.

In an additional and/or alternative embodiment, the genetically engineered microbial cell possesses a UDP-*N*-acetylglucosamine biosynthesis pathway, e.g. comprising a L-glutamine:D-fuctose-6-phosphate aminotransferase, a phosphoglucosamine mutase and a *N*-acetylglucosamine-1-phosphate uridyltransferase/ glucosamine-1-phosphate acetyltransferase.

The intracellular supply of UDP-*N*-acetylglucosamine can be improved by genetic modifications such as an expression or overexpression of one or more of the genes encoding polypeptides exhibiting L-glutamine:D-fuctose-6-phosphate aminotransferase activity (e.g. the *E. coli* K-12 *glmS* gene (acc. no. NP_418185)), phosphoglucosamine mutase activity (e.g. the *E. coli* K-12 *glmM* gene (acc. no. NP_417643)) and *N*-acetylglucosamine-1-phosphate uridyltransferase/ glucosamine-1-phosphate acetyltransferase activity (e.g. the *E*. *coli* K-12 *glmU* gene (acc. no. NP_418186)), or variants thereof.

In another embodiment, the genetically engineered microbial cell possesses an CMP-*N*-acetylneuraminic acid/CMP-sialic acid biosynthesis pathway for intracellular formation of CMP-*N*-acetylneuraminic acid (CMP-Neu5Ac), required for sialyltransferase reactions leading e.g. to the formation of sialylated di- or oligosaccharides.

In an additional and/or alternative embodiment, the genetically engineered microbial cell possesses a CMP-*N*-acetylneuraminic acid biosynthesis pathway, e.g. comprising a L-glutamine:D-fuctose-6-phosphate aminotransferase, a phosphoglucosamine mutase, a *N*-acetylglucosamine-1-phosphate uridyltransferase/ glucosamine-1-phosphate acetyltransferase, UDP-N-acetylglucosamine-2-epimerase, a glucosamine-6-phosphate acetyltransferase, a *N*-acetylglucosamine-6-phosphate phosphatase (preferably a HAD-like sugar phosphatase), a *N*-acetylglucosamine 2-epimerase, a sialic acid synthase and a CMP-sialic acid synthetase.

The intracellular supply of CMP-*N*-acetylneuraminic acid can be improved by genetic modifications such as an expression or overexpression of one or more of the genes encoding polypeptides exhibiting L-glutamine:D-fuctose-6-phosphate aminotransferase activity (e.g. the *E. coli* K-12 *glmS* gene), phosphoglucosamine mutase activity (e.g. the *E. coli* K-12 *glmM* gene), *N*-acetylglucosamine-1-phosphate uridyltransferase/ glucosamine-1-phosphate acetyltransferase activity (e.g. the E. *coli* K-12 *glmU* gene), UDP-*N*-acetylglucosamine-2-epimerase activity (e.g. the *Campylobacter jejuni neuC* gene (acc. no. AF305571)), a glucosamine-6-phosphate acetyltransferase activity (e.g. the *Saccharomyces cerevisiae gna1* gene (acc. no. NP_116637)), a *N*-acetylglucosamine-6-phosphate phosphatase activity (e.g. the *E*. *coli* K-12 *yihXgene* (acc. no. NP_418321)), a *N-*acetylglucosamine 2-epimerase activity (e.g. the *Synechocystis sp.* PCC6803 *slr1975* gene (acc. no. BAL35720)), a sialic acid synthase activity (e.g. the *Campylobacter jejuni neuB* gene (acc. no. AF305571)) and a CMP-sialic acid synthetase activity (e.g. the *Campylobacter jejuni neuA* gene (acc. no. AF305571)), or variants thereof.

The genetically engineered microbial cell may further comprise a glycosyltransferase. In a preferred embodiment, at least one glycosyltransferase is a fucosyltransferase, a sialyltransferase, a glucosaminyltransferase or a galactosyltransferase, more preferably, the at least one glycosyltransferase exhibits β-1,3-galactosyltransferase activity, β-1,4-galactosyltransferase activity, β-1,6-galactosyltransferase activity, β-1,3-*N*-acetylglucosaminyltransferase activity, α-2,3-sialyltransferase activity, α-2,6-sialyltransferase activity, α-1,2-fucosyltransferase activity, α-1,3-fucosyltransferase activity, α-1,4-fucosyltransferase activity. Suitable glycosyltransferases are known to the skilled artisan or can be found in the literature.

Generally, and throughout the present disclosure, the term "glycosyltransferase activity" or "glycosyltransferase" designates and encompasses enzymes that are responsible for the biosynthesis of disaccharides, oligosaccharides and polysaccharides, and they catalyze the transfer of monosaccharide moieties from an activated nucleotide monosaccharide/sugar (e.g. UDP-Glc, UDP-Gal, GDP-Fuc, UDP-GlcNAc, CMP-Neu5Ac) to a glycosyl acceptor molecule (e.g. mono-, di- or oligosaccharides).

In an additional and/or alternative embodiment, the genetically engineered microbial cell synthesizes more phosphoenolpyruvate (PEP) than the wildtype of the cell. In an additional and/or alternative embodiment, the genetically engineered microbial cell has been genetically engineered to possess an enhanced PEP biosynthesis pathway. For example, the genetically engineered microbial cell has been genetically engineered to possess an increased phosphoenolpyruvate carboxykinase activity, e.g. by overexpressing the *E*. *coli* K-12 *pckA* gene (acc. no. NP_417862) or a functional variant thereof. Preferably, the genetically engineered host cell has been genetically engineered to possess an increased phosphoenolpyruvate synthase activity, for example in that the *E*. *coli* K-12 *ppsA* gene (acc. no. NP_416217) encoding phosphoenolpyruvate synthase is overexpressed and/or in that the nonnaturally-occurring microorganisms contains at least one additional copy of a nucleotide sequence allowing the expression of a phosphoenolpyruvate synthase or a functional variant thereof. Overexpression of *pckA or ppsA* enhances intracellular PEP synthesis such that more PEP is available, e.g. for the production of sialic acid.

In an additional and/or alternative embodiment, the intracellular PEP-availability of the genetically engineered host cell can be improved by decreasing and/or diminishing the activity of a phosphoenolpyruvate:sugar phosphotransferase dependent importer required for the transfer of a certain sugar (e.g. glucose), used as carbon- and energy source, from the culture medium into the cell. Consequently, in order to maintain the capability of the genetically engineered host cell to use said certain sugar as carbon- and energy source, a phosphoenolpyruvate:sugar phosphotransferase independent importer needs to be expressed or overexpressed. In an additional and/or alternative embodiment, the expression or overexpression of a kinase, capable to phosphorylate said certain sugar, is required or decreased and/or diminished, in order to realize metabolization of said sugar or its accumulation in an unphosphorylated form within the cell, respectively.

Phosphoenolpyruvate:sugar phosphotransferase dependent importer or components thereof, whose expression and/or activity might be decreased and/or diminished in the genetically engineered microbial cell (e.g. E. *coli* K-12) comprise at least one selected from the group consisting of the glucose PEP-PTS genes *ptsG* (acc. no. NP_415619), *malX* (acc. no. NP_416138), crr (acc. no. NP_416912), *bglF* (acc. no. NP_418178) and/or the fructose PEP-PTS genes *fruA* (acc. no. NP_416672), *fruB* (acc. no. NP_416674) and/or the mannose PEP-PTS genes *manX* (acc. no. NP_416331), *many* (acc. no. NP_416332), *manZ* (acc. no. NP_416333) and or the *N*-acetylglucosamine PEP-PTS gene *nagE* (acc. no. NP_415205).

Suitable non-PEP-PTS transporter or variants thereof, capable of transferring monosaccharides (glucose and/or fructose and/or galactose and/or N-acetylglucosamine and/or *N*-acetylneuraminic acid and/or fucose) into a microbial cell, are known to the skilled artesian. Non-limiting examples of genes encoding said non-PEP-PTS transporter are *galP* of *Escherichia coli* K-12 (SEQ ID NO. 1), *glf of Zymomonas mobilis* (SEQ ID NO. 2), *cscB* of *Escherichia coli W* (SEQ ID NO. *3), fupL* of *Leuconostoc pseudomesenteroides* (SEQ ID NO. 4), *lac Y* of *Escherichia coli* K-12 SEQ ID NO. 5), *fucP* of *Escherichia coli* K-12 (SEQ ID NO. 6), *nanT* of *Escherichia coli* K-12 (SEQ ID NO. 7), *nagP* of *Xanthomonas campestris* (SEQ ID NO. 8), *glcP* of *Bifidobacterium longum* NCC2705 (SEQ ID NO. 9), *glcP* of *Bacillus subtilis* (SEQ ID NO. 10), *sglS* of *Vibrio parahaemolyticus* (SEQ ID NO. 11), *xylE* of *Escherichia coli* K-12 (SEQ ID NO. 12), *araE* of *Bacillus subtilis* 168 (SEQ ID NO. 13). Thus, in an additional and/or alternative embodiment, the genetically engineered host cell comprises and expresses at least one gene comprising the protein coding region of the aforementioned genes or functional variants thereof.

In a preferred embodiment, suitable non-PEP-PTS-glucose transporter are a sugar facilitated diffusion protein and/or a glucose translocation permease. A suitable glucose facilitated fusion protein is encoded by the *glf* gene of *Zymomonas mobilis.* A suitable glucose translocation permease is encoded by the *E*. *coli* K-12 *galP* gene. The glucose translocation permease is also known as galactose-proton symporter or galactose premease, but also imports glucose across the cell membrane.

In another preferred embodiment, suitable non-PEP-PTS-fructose transporter are a sugar facilitated diffusion protein and/or a fructose carrier protein. A suitable fructose facilitated fusion protein is encoded by the *glf* gene of *Zymomonas mobilis.* A suitable fructose carrier protein is encoded by the *Leuconostoc pseudomesenteroides fupL* gene.

Generally, and throughout the present disclosure the terms "increased intracellular availability of a sugar phosphate" or "increased/improved supply of a sugar phosphate" refer to an increased capability of the genetically modified microbial cell to generate elevated intracellular amounts of said sugar phosphate, compared to an unmodified microbial cell, enabling an elevated carbon flux through the biosynthesis pathway of a desired carbohydrate. Consequently, an elevated production of said desired carbohydrate by said genetically modified microbial cell is a direct measure of an elevated intracellular amount of said sugar phosphate. Besides, methods targeting the intracellular metabolite quantification and/or the carbon flux of cells can be found in the literature known to the skilled artisan (e.g. Lämmerhofer and Weckwerth, "Metabolomics in Practice: Successful Strategies to Generate and Analyze Metabolic Data", Wiley-VCH, Weinheim, Germany (2013).

In an additional and/or alternative embodiment, the expression and/or activity of genes and/or proteins, respectively, competing with the biosynthesis of the desired carbohydrate may be decreased and/or diminished within the genetically engineered host cell. Non-limiting examples of such counteracting proteins/protein activities comprise at least one selected from the group consisting of β-galactosidase (e.g. *E*. *coli K-12* LacZ (acc. no. NP_414878)), UDP-glucose:undecaprenyl-phosphate glucose-1-phosphate transferase (e.g. *E.coli* K-12 WcaJ (acc. no. NP_416551)), L-fucose isomerase (e.g. *E*. *coli* K-12 Fucl), fuculokinase (e.g. *E*. *coli* K-12 FucK (acc. no. NP_417282)), *N*-acetylglucosamine-6-phosphate deacetylase (e.g. *E*. *coli* K-12 NagA (acc. no. NP_415203)), glucosamine-6-phosphate deaminase (e.g. *E*. *coli K-12* NagB (acc. no. NP_415204)), *N*-acetylmannosamine kinase (e.g. *E*. *coli K-12* NanK (acc. no. NP_417689)), *N*-acetylmannosamine-6-phosphate epimerase (e.g. *E*. *coli* K-12 NanE (acc. no. NP_417690)), N-acetylneuraminic acid aldolase (e.g. *E*. *coli* K-12 NanA (acc. no. NP_417692)), sialic acid permease (e.g. *E*. *coli K-12* NanT (acc. no. NP_417691)).

In an additional and/or alternative embodiment, the genetically engineered microbial cell is capable to metabolize sucrose, thus, comprising one or more genes encoding (i) a heterologous PTS-dependent sucrose utilization transport system (e.g. the *Klebsiella pneumoniae scrYAB* genes (SEQ ID NO. 14) or the *Salmonella typhimurium scrYAB* genes (SEQ ID NO. 15) consisting of a sucrose porin (*scrY*)*,* a PTS sucrose-specific transporter subunit II (*scrA*) and a sucrose-6-phosphate hydrolase (scr*B*) and/or (ii) a heterologous PTS-dependent sucrose transport system (e.g. the *Klebsiella pneumoniae scrYA* genes or the *Salmonella typhimurium scrYA* genes) in combination with a sucrose phosphate synthase gene (e.g. the *Anabaena sp.* PCC7120 *spsA* gene (acc. no. AJ302071)) and/or (iii) a heterologous PTS-independent sucrose utilization system (e.g. the *E*. *coli* W *cscBKA* genes (SEQ ID NO. 16) consisting of a fructokinase (*cscK*)*,* a sucrose hydrolase (*cscA*) and a sucrose permease (csc*B*) and/or (iv) a heterologous sucrose utilization system consisting of a sucrose permease gene (e.g. *E*. *coli* W *cscB*) in combination with a sucrose phosphorylase gene (e.g. the *Bifidobacterium adolescentis basP* gene (acc. no. WP_011742626)) or a sucrose synthase gene (e.g. the *Anabaena sp. susA* gene (acc. no. CAA09297)).

In an additional and/or alternative embodiment, the microbial cell comprises an exporter protein or a permease exporting the carbohydrate of interest from the cell, preferably a sugar efflux transporter.

In an additional and/or alternative embodiment, the host cell is a microbial cell, preferably a bacterial cell selected from the group consisting of bacteria of the genera *Escherichia, Lactobacillus, Corynebacterium, Bacillus, Streptococcus, Enterococcus, Lactococcus* and *Clostridium,* preferably a bacterial cell that is selected from the group of bacterial species consisting of *Escherichia coli, Corynebacterium glutamicum, Clostridium cellulolyticum, Clostridium Ijungdahlii, Clostridium autoethanogenum, Clostridium acetobutylicum, Bacillus subtilis, Bacillus megaterium, Lactobacillus casei, Lactobacillus acidophilus, Lactobacillus helveticus, Lactobacillus delbrueckii,* and *Lactococcus lactis.* In another embodiment, the microbial cell is an *Escherichia coli.* A person skilled in the art will be aware of further bacterial strains when reading the present disclosure.

According to the second aspect, provided is the use of a genetically engineered host cell as described herein for the production of a desired carbohydrate, wherein said host cell is cultivated in the presence of a monosaccharide mixture consisting of glucose and at least a second monosaccharide of the group of fructose and galactose. Said cell is genetically engineered to exhibit an increased intracellular availability of at least one sugar phosphate relevant for the production of the desired carbohydrate. Said sugar phosphate may be selected from the group consisting of phosphoenolpyruvate (PEP), dihydroxyacetone phosphate, fructose-6-phosphate, fructose-1-phosphate, fructose-1,6-bisphosphate, glucose-6-phosphate, glucose-1-phosphate, galactose-1-phosphate, mannose-1-phosphate, mannose-6-phosphate, glucosamine-6-phosphate, glucosamine-1-phosphate, N-acetylglucosamine-6-phosphate, *N*-acetylglucosamine-1-phosphate, N-acetylmannosamine-6-phosphate, UDP-glucose, UDP-galactose, CMP-N-acetylneuraminic acid, GDP-mannose, GDP-fucose and UDP-N-acetylglucosamine.

According to the third aspect, provided is a method for the fermentative production of a desired carbohydrate comprising the steps of:
a) providing a genetically engineered microorganism capable of producing a desired carbohydrate, wherein said microorganism is exhibiting an increased intracellular availability of at least one sugar phosphate due to a decreased and/or diminished expression and/or activity of at least one protein leading to a consumption of said intracellular sugar phosphate within said engineered microorganism;
b) cultivating said genetically engineered microorganism in a culture medium permissive for the production of said desired carbohydrate, wherein the main carbon source is a monosaccharide mixture consisting of glucose and at least a second monosaccharide of the group of fructose and galactose;
c) recovering said desired saccharide from the culture broth.

In an additional and/or alternative embodiment, the carbohydrate of interest is a human milk oligosaccharide or a building block thereof. A list of carbohydrates of interest that can be produced by using a genetically modified microbial cell and/or a method as described herein is disclosed in table 1. Preferably, the desired carbohydrate is selected from the group consisting of 2'-fucosyllactose, 3-fucosyllactose, 2',3-difucosyllactose, 3'-sialyllactose, 6'-sialyllactose, 3-fucosyl-3'-sialyllactose, lacto-*N*-tetraose, lacto-*N*-neotetraose, lacto-*N*-fucopentaose I, lacto-N-fucopentaose II, lacto-*N*-fucopentaose III, lacto-*N*-fucopentaose V, lacto-N-difucosylhexose I, lacto-*N*-difucosylhexaose II, lacto-*N*-sialylpentaose LSTa, LSTb, LSTc.

Preferably, the mixture of glucose and at least one additional monosaccharide is a mixed feedstock of glucose and fructose, preferably obtained by hydrolyzation of sucrose.

In an additional and/or alternative embodiment, sucrose hydrolyzation is incomplete, such that substantial amounts of sucrose remain in the feedstock. Thus, the sucrose amount in the hydrolyzed and/or heat-sterilized feedstock is greater than 5%, 10%, 15%, 20%, 25%, 30%, 35%, 40%, 45%, 50%, 55%, 60%, 65 %, 70%, 75% or greater than 80%.

In an additional and/or alternative embodiment, the microbial cell is cultivated without exogenous supply of an acceptor substrate, e.g. *N*-acetylglucosamine or lactose, in particular when cultivated for the production of the oligosaccharide of interest.

The present invention will be described with respect to particular embodiments and with reference to drawings, but the invention is not limited thereto but only by the claims. Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description and drawings provided herein, numerous specific details are set forth. However, it is understood that embodiments of the invention may be practiced without these specific details. In other instances, well-known methods, structures and techniques have not been shown in detail in order not to obscure an understanding of this description.

**Table 1: Non-limiting list of carbohydrates of interest that can be produced by using a genetically modified microbial cell and/or a method as described herein.**

| Name | Abbrev. | structure |
|---|---|---|
| Mannose | Man | Man |
| Fucose | Fuc | Fuc |
| *N*-acetylglucosamine | GlcNAc | GlcNAc |
| *N-*acetylmannosamine | ManNAc | ManNAc |
| *N*-acetylneuraminic acid | Neu5Ac | Neu5Ac |
| Lacto-*N*-biose | LNB | Gal(β1-3)GlcNAc |
| *N*-acetyllactosamine | LacNAc | Gal(β1-4)GlcNAc |
| 3-Fucosylglucose | Fuc-Glc | 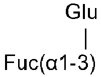 |
| 4'-Fucosyl-*N-*acetylglucosamine | Fuc-GlcNAc | 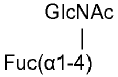 |
| 6'-Sialyl-N-acetylglucosamine | Neu5Ac-GlcNAc | 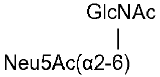 |
| 2'-Fucosyllactose | 2'-FL | Fuc(α1-2)Gal((β1-4)Glu |
| 3-Fucosyllactose | 3-FL | 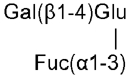 |
| 2',3-Difucosyllactose | DF-L | 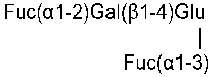 |
| Blood group H antigen type I | HA-T I | Fuc(α1-2)Gal((β-3)GlcNAc |
| Blood group H antigen type II | HA-T II | Fuc(α1-2)Gal((β1-4)GlcNAc |
| Blood group antigen Lewis^{a} | Le^{a} | 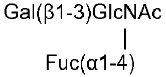 |
| Blood group antigen Lewis^{b} | Le^{b} | 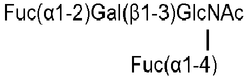 |
| Blood group antigen Lewis^{x} | Le^{x} | 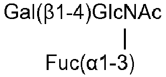 |
| Blood group antigen Lewis^{y} | Le^{y} | 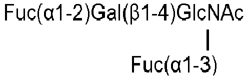 |
| Blood group antigen Sialyl-Lewis^{x} | SLe^{x} | 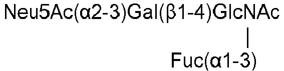 |
| Lacto-*N*-triose II | LNT II | GlcNAc(β1-3)Gal(β1-4)Glu |
| Lacto-*N*-tetraose | LNT | Gal(β1-3)GlcNAc(β1-3)Gal(β1-4)Glu |
| Lacto-*N*-neotetraose | LNnT | Gal(β1-4)GlcNAc(β1-3)GalGal(β1-4)Glu |
| Lacto-*N-*fucopentaose I | LNFP I | Fuc(α1-2)Gal(Gal(β1-3)GlcNAc(Gal(β1-3)Gal(Gal(β1-4)Glu |
| Lacto-*N-*neofucopentaose I | LNnFP I | Fuc(α1-2)Gal(Gal(β1-4)GlcNAc(Gal(β1-3)Gal(Gal(β1-4)Glu |
| Lacto-*N-*fucopentaose II | LNFP II | 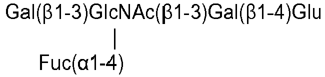 |
| Lacto-*N-*fucopentaose III | LNFP III | 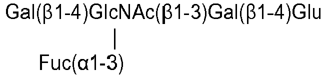 |
| Lacto-*N-*fucopentaose V | LNFP V | 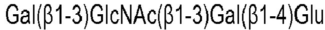 |
| | | 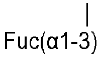 |
| Lacto-*N-*neofucopentaose V | LNnFP V | 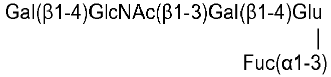 |
| Lacto-*N-*difucohexaose I | LNDH I | 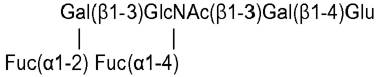 |
| Lacto-*N-*difucohexaose II | LND | 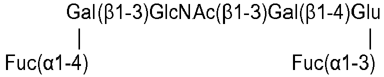 |
| 6'-Galactosyllactose | 6'-GL | Gal(β1-6)Gal(β1-4)Glu |
| 3'-Galactosyllactose | 3'-GL | Gal(β1-3)Gal(β1-4)Glu |
| Lacto-*N*-hexaose | LNH | 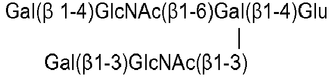 |
| Lacto-*N*-neohexaose | LNnH | 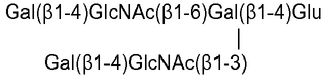 |
| *para*-Lacto-*N-*hexaose | paraLNT | Gal(β1-3)GlcNAc(β1-3)Gal(β1-4)GlcNAc(β1-3)Gal(β1-4)Glu |
| *para*-Lacto-*N-*neohexaose | paraLNnH | Gal(β1-4)GlcNAc(β1-3)Gal(β1-4)GlcNAc(β1-3)Gal(β1-4)Glu |
| Difucosyl-lacto-*N-ne*ohexaose | DF-LNnH | 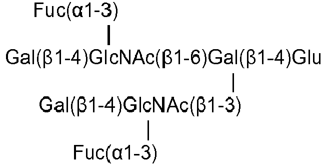 |
| 3'-Sialyllactose | 3'-SL | Neu5Ac(α2-3)Gal(β1-4)Glu |
| 6'-Sialyllactose | 6'-SL | Neu5Ac(α2-6)Gal((β1-4)Glu |
| 3'-Sialyl-lacto-*N-*biose | 3'-SLNB | Neu5Ac(α2-3)Gal(β1-3)GlcNAc |
| 6'-Sialyl-lacto-*N-*biose | 6'-SLNB | Neu5Ac(α2-6)Gal((β1-3)GlcNAc |
| 3'-Sialyl-*N-*acetyllactosamine | 3'-SLN | Neu5Ac(α2-3)Gal(β1-4)GlcNAc |
| 6'-Sialyl-N-acetyllactosamine | 6'-SLN | Neu5Ac(α2-6)Gal(β1-4)GlcNAc |
| Lacto-*N-*sialylpentaose a | LST-a | Neu5Ac(α2-3)Gal(β1-3)GlcNAc(β1-3)Gal(β1-4)Glu |
| Lacto-*N-*sialylpentaose b | LST-b | 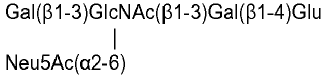 |
| Lacto-*N-*sialylpentaose c | LST-c | Neu5Ac(α2-6)Gal(β1-4)GlcNAc(β1-3)Gal((β1-4)Glu |
| Fucosyl-lacto-*N-*sialylpentaose a | F-LST-a | 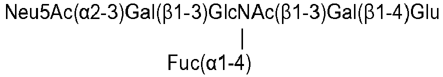 |
| Fucosyl-lacto-*N-*sialylpentaose b | F-LST-b | 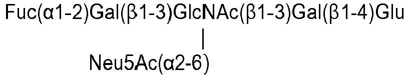 |
| Fucosyl-lacto-*N-*sialylpentaose c | F-LST-c | 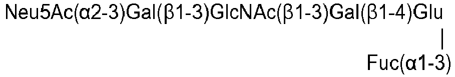 |
| Disialyl-lacto-*N-*tetraose | DS-LNT | 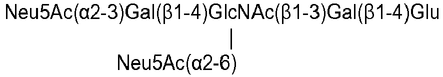 |
| Disialyl-lacto-*N-*fucopentaose | DS-LNFP V | 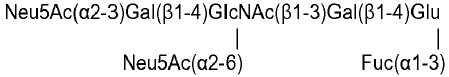 |
| 3-Fucosyl-3'-sialyllactose | 3F-3'-SL | 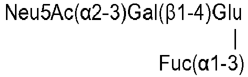 |
| 3-Fucosyl-6'-sialyllactose | 3F-6'-SL | 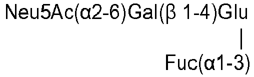 |
| Lacto-*N-ne*odifucohexaose I | LNnDFH I | 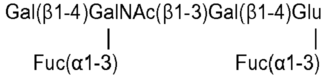 |

The invention will now be described by a detailed description of several embodiments of the invention. It is clear that other embodiments of the invention can be configured according to the knowledge of persons skilled in the art without departing from the true spirit or technical teaching of the invention, the invention being limited only by the terms of the appended claims.

In an embodiment an *E*. *coli* strain possessing the genotype *nagABE⁻, manXYZ⁻* is metabolically engineered as an efficient producer of *N*-acetylglucosamine (GlcNAc) using a mixed-monosaccharide feedstock (e.g. hydrolyzed sucrose) as main carbon- and energy source. Therefore, the heterologous expression of a gene encoding for a glucosamine-6-phosphate acetyltransferase, capable to transfer acetate from acetyl-CoA to glucosamine-6-phosphate, thus generating N-acetylglucosamine-6-phosphate, is necessary. In a preferred embodiment, this production strain is further genetically engineered by decreasing and/or diminishing the expression of the phosphofructokinase genes *pfkA* and/or *pfkB* and/or the glucose-6-phosphate dehydrogenase gene *zwf*. This further genetic modification allows cultivation of the engineered production strain on a mixed-monosaccharide feedstock (e.g. hydrolyzed sucrose) as main carbon- and energy source, while preventing to hamper the strain's metabolism but increasing precursor supply (fructose-6-phosphate) for *N*-acetylglucosamine production. In an additional embodiment a gene encoding for a glutamine-fructose-6-phosphate aminotransferase (e.g. *E. coli* GlmS) and/or a gene encoding for a HAD-like sugar phosphatase (e.g. *E. coli* YihX, *E*. *coli* YqaB), capable to dephosphorylate N-acetylglucosamine-6-phosphate to *N*-acetylglucosamine, is expressed/ overex-pressed in order to foster the synthesis of GlcNAc.

In another embodiment, an *E*. *coli* strain possessing the genotype *lacY⁺, lacZ⁻, fuclK⁻, wcaJ⁻* is metabolically engineered as an efficient producer of L-fucose using a mixed-monosaccharide feedstock (e.g. hydrolyzed sucrose) as main carbon- and energy source as well as lactose as acceptor substrate. Therefore, the overexpression of at least one of the *E*. *coli* genes *manA*, *manC*, *manB*, *gmd* and *wcaG* as well as the expression of a heterologous α-1,2-fucosyltransferase, capable to transfer fucose from GDP-fucose to lactose, thus generating 2'-fucosyllactose, and an α-1,2-fucosidase, capable to release free L-fucose from 2'-fucosyllactose, is necessary. In a preferred embodiment, this production strain is further engineered by decreasing and/or diminishing the expression of the phosphofructokinase genes *pfkA* and/or *pfkB* and/or the glucose-6-phosphate dehydrogenase gene *zwf.* This further genetic modification allows cultivation of the engineered production strain on a mixed-monosaccharide feedstock (e.g. hydrolyzed sucrose) as main carbon- and energy source, while preventing to hamper the strain's metabolism but increasing precursor supply (fructose-6-phosphate) for L-fucose production.

In another embodiment an *E*. *coli* strain possessing the genotype *nanKETA⁻, nagAB⁻* is metabolically engineered as an efficient producer of *N*-acetylneuraminic acid (Neu5Ac) using a mixed-monosaccharide feedstock (e.g. hydrolyzed sucrose) as main carbon- and energy source. Therefore, the overexpression of at least one of the genes encoding for either (i) a glucosamine-6-phosphate acetyltransferase and a *N*-acetylglucosamine-2 epimerase and a *N*-acetylneuraminic acid synthetase, or (ii) a UDP-*N*-acetylglucosamine 2-epimerase and a *N-*acetylneuraminic acid synthetase, is necessary. Since the *N*-acetylneuraminic acid synthesis is a phosphoenolpyruvate (PEP)-dependent process, competing reactions leading to PEP consumption are preferably avoided. Thus, in a preferred embodiment, this production strain is further engineered by decreasing and/or diminishing the import of said carbon- and energy source(s) through a phosphoenolpyruvate:sugar phosphotransferase dependent mechanism, e.g. by decreasing and/or diminishing the expression of the glucose PEP permease gene *ptsG* and/or the fructose PEP permease gene *fruA* and/or the mannose PEP permease genes *manXYZ.* Along with the expression/overexpression of at least one gene encoding for a phosphoenolpyruvate:sugar phosphotransferase independent (non-PEP-PTS) transporter, enabling monosaccharide(s) transfer into the engineered cell, as well as a fructose kinase (e.g. the *E. coli* W *cscK* gene) and a glucose kinase (e.g. the *E*. *coli* K-12 *glk* gene), capable to activate fructose to fructose-6-phosphate and glucose to glucose-6-phosphate, respectively, this further genetic modification allows the cultivation of the engineered production strain on a mixed-monosaccharide feedstock (e.g. hydrolyzed sucrose) as main carbon- and energy source, while preventing to hamper the strain's metabolism but increasing precursor supply (fructose-6-phosphate, phosphoenolpyruvate) for N-acetylneuraminic acid production.

In another embodiment an *E*. *coli* strain possessing the genotype *nagABE⁻, manXYZ⁻, lacZ⁻* is metabolically engineered as an efficient producer of N-acetyllactosamine (LacNAc) using a mixed-monosaccharide feedstock (e.g. hydrolyzed sucrose) as main carbon- and energy source as well as GlcNAc as acceptor substrate. Therefore, the expression/overexpression of genes encoding for a glucosamine-6-phosphate acetyltransferase, a phosphoenolpyruvate:sugar phosphotransferase independent transporter, capable to transfer N-acetylglucosamine into the cell, and a β-1,4-galactosyltransferase, capable to transfer galactose from UDP-Gal to free *N*-acetylglucosamine, thus generating N-acetyllactosamine, is necessary. In a preferred embodiment, this production strain is further genetically engineered by decreasing and/or diminishing the expression of the phosphofructokinase genes *pfkA* and/or *pfkB* and/or the glucose-6-phosphate isomerase gene *pgi* and/or the glucose-6-phosphate dehydrogenase gene *zwf.* This further genetic modification allows cultivation of the engineered production strain on a mixed-monosaccharide feedstock (e.g. hydrolyzed sucrose) as main carbon- and energy source, while preventing to hamper the strain's metabolism but increasing precursor supply (glucose-6-phosphate) for *N*-acetyllactosamine production. In an additional embodiment at least one of the *E. coli* genes *pgm, galU* and *galE* is overexpressed in order to foster the synthesis of UDP-Gal.

In another embodiment an *E. coli* strain possessing the genotype *nagAB⁻* is metabolically engineered as an efficient producer of lacto-*N*-biose (LNB) by means of total fermentation using a mixed-monosaccharide feedstock (e.g. hydrolyzed sucrose) as main carbon- and energy source. Therefore, the expression/ overexpression of genes encoding for a glucosamine-6-phosphate acetyltransferase, capable to transfer acetate from acetyl-CoA to glucosamine-6-phosphate, thus generating *N*-acetylglucosamine-6-phosphate, a HAD-like sugar phosphatase, capable to dephosphorylate *N*-acetylglucosamine-6-phosphate, thus generating *N*-acetylglucosamine, and a β-1,3-galactosyltransferase, capable to transfer galactose from UDP-Gal to free *N*-acetylglucosamine, thus generating lacto-N-biose, is necessary. In a preferred embodiment, this production strain is further genetically engineered by decreasing and/or diminishing the expression of the phosphofructokinase genes *pfkA* and/or *pfkB* and/or the glucose-6-phosphate dehydrogenase gene *zwf.* This further genetic modification allows cultivation of the engineered production strain on a mixed-monosaccharide feedstock (e.g. hydrolyzed sucrose) as main carbon- and energy source, while preventing to hamper the strain's metabolism but increasing precursor supply (fructose-6-phosphate and glucose-6-phosphate) for lacto-*N*-biose production. In an additional embodiment at least one of the *E. coli* genes *glmS, pgm, galU, galE* is overexpressed in order to foster the synthesis of GlcNAc and/or UDP-Gal.

In another embodiment an *E*. *coli* strain possessing the genotype *lacY⁺*, *lacZ⁻*, *nanKETA⁻*, *nagAB⁻* is metabolically engineered as an efficient producer of 3'-sialyllactose using a mixed-monosaccharide feedstock (e.g. hydrolyzed sucrose) as main carbon- and energy source as well as lactose as acceptor substrate. Therefore, an *N*-acetylneuraminic acid production strain, as described, is genetically engineered by the expression of a heterologous CMP-N-acetylneuraminic acid synthetase, a *N*-acetylneuraminic acid synthase and an α-2,3-sialyltransferase, capable to transfer *N*-acetylneuraminic acid from CMP-Neu5Ac to lactose, thus generating 3'-sialyllactose. As for the synthesis of N-acetylneuraminic acid, 3'-SL production is a phosphoenolpyruvate (PEP)-dependent process. Thus, in a preferred embodiment, said 3'-SL production strain is further engineered by decreasing and/or diminishing the import of said carbon- and energy source(s) through a phosphoenolpyruvate:sugar phosphotransferase dependent mechanism, e.g. by decreasing and/or diminishing the expression of the glucose PEP permease gene *ptsG* and/or the fructose PEP permease gene *fruA* and/or the mannose PEP permease genes *manXYZ*. Along with the expression/overexpression of at least one gene encoding for a phosphoenolpyruvate:sugar phosphotransferase independent (non-PEP-PTS) transporter, enabling monosaccharide(s) transfer into the engineered cell, as well as a fructose kinase (e.g. the *E*. *coli W cscK* gene) and a glucose kinase (e.g. the *E*. *coli* K-12 *glk* gene), capable to activate fructose to fructose-6-phosphate and glucose to glucose-6-phosphate, respectively, this further genetic modification allows the cultivation of the engineered production strain on a mixed-monosaccharide feedstock (e.g. hydrolyzed sucrose) as main carbon- and energy source, while preventing to hamper the strain's metabolism but increasing precursor supply (fructose-6-phosphate, phosphoenolpyruvate) for 3'-sialyllactose production.

In another embodiment an *E. coli* strain possessing the genotype *lacY⁺*, *lacZ⁻*, *fuclK⁻*, *wcaJ⁻* is metabolically engineered as an efficient producer of 3-fucosyllactose using a mixed-monosaccharide feedstock (e.g. hydrolyzed sucrose) as main carbon- and energy source as well as lactose as acceptor substrate. Therefore, the overexpression of at least one of the *E. coli* genes *manA, manC, manB, gmd* and *wcaG* as well as the expression of a heterologous α-1,3-fucosyltransferase, capable of transferring fucose from GDP-fucose to lactose, thus generating 3-fucosyllactose, is necessary. In a preferred embodiment, this production strain is further genetically engineered by decreasing and/or diminishing the expression of the phosphofructokinase genes *pfkA* and/or *pfkB* and/or the glucose-6-phosphate dehydrogenase gene *zwf.* This further genetic modification allows cultivation of the engineered production strain on a mixed-monosaccharide feedstock (e.g. hydrolyzed sucrose) as main carbon- and energy source, while preventing to hamper the strain's metabolism but increasing precursor supply (fructose-6-phosphate) for 3-fucosyllactose production.

In one embodiment an *E. coli* strain possessing the genotype *lacY⁺*, *lacZ⁻*, *nagB⁻*, *wcaJ⁻* is metabolically engineered to efficiently produce lacto-*N*-triose II (LNT-II) using a mixed-monosaccharide feedstock (e.g. hydrolyzed sucrose) as main carbon- and energy source as well as lactose as acceptor substrate. Therefore, the expression of a heterologous β-1,3-*N*-acetylglucosaminyltransferase, capable to transfer *N*-acetylglucosamine from UDP-GlcNAc to lactose, thus generating lacto-N-triose II, is necessary. In a preferred embodiment, this production strain is further genetically engineered by decreasing and/or diminishing the expression of the phosphofructokinase genes *pfkA* and/or *pfkB* and/or the glucose-6-phosphate dehydrogenase gene *zwf.* This further genetic modification allows cultivation of the engineered production strain on a mixed-monosaccharide feedstock (e.g. hydrolyzed sucrose) as main carbon- and energy source, while preventing to hamper the strain's metabolism but increasing precursor supply (fructose-6-phosphate) for lacto-*N*-triose II production. In an additional embodiment at least one of the *E. coli* genes *glmS, glmU* and *glmM* is overexpressed in order to foster UDP-GlcNAc synthesis.

In another embodiment an *E. coli* strain possessing the genotype *lacY⁺, lacZ⁻, nagB⁻, wcaJ⁻* is metabolically engineered as an efficient producer of lacto-N-tetraose (LNT) using a mixed-monosaccharide feedstock (e.g. hydrolyzed sucrose) as main carbon- and energy source as well as lactose as acceptor substrate. Therefore, the expression of a heterologous β-1,3-*N-*acetylglucosaminyltransferase, capable to transfer *N*-acetylglucosamine from UDP-GlcNAc to lactose, and a β-1,3-galactosyltransferase, capable to transfer galactose from UDP-galactose to lacto-*N*-triose II, thus generating lacto-*N*-tetraose, is necessary. In a preferred embodiment, this production strain is further genetically engineered by decreasing and/or diminishing the expression of the phosphofructokinase genes *pfkA* and/or *pfkB* and/or the glucose-6-phosphate dehydrogenase gene *zwf* and/or the glucose-6-phosphate isomerase gene *pgi.* This further genetic modification allows cultivation of the engineered production strain on a mixed-monosaccharide feedstock (e.g. hydrolyzed sucrose) as main carbon- and energy source, while preventing to hamper the strain's metabolism but increasing precursor supply (fructose-6-phosphate and glucose-6-phosphate) for lacto-*N*-tetraose production. In an additional embodiment at least one of the *E. coli* genes *glmS, glmU, glmM, pgm, galU* and *galE* is overexpressed in order to foster the synthesis of UDP-GlcNAc and/or UDP-Gal.

In another embodiment an *E. coli* strain possessing the genotype *lacY⁺, lacZ⁻, nagB⁻, wcaJ⁻* is metabolically engineered to efficiently produce lacto-*N*-neotetraose (LNnT) by means of total fermentation using a mixed-monosaccharide feedstock (e.g. hydrolyzed sucrose) as main carbon- and energy source. Therefore, the LNnT production strain is genetically engineered by decreasing and/or diminishing the import of glucose through a phosphoenolpyruvate:sugar phosphotransferase dependent mechanism, e.g. by decreasing and/or diminishing the expression of the glucose PEP permease gene *ptsG,* while expressing at least one gene encoding for a phosphoenolpyruvate:sugar phosphotransferase independent (non-PEP-PTS) transporter, enabling glucose transfer into the engineered cell. Furthermore, the expression of the glucokinase gene *glk* and/or the glucose dehydrogenase gene *gcd* is decreased and/or abolished. Essentially, the heterologous expression of a β-1,4-galactosyltransferase, capable to transfer galactose from UDP-galactose on glucose, thus generating lactose, and a β-1,3-*N*-acetylglucosaminyltransferase, capable of transferring *N*-acetylglucosamine from UDP-GlcNAc to lactose, thus generating lacto-*N*-triose II, and a β-1,4-galactosyltransferase, capable to transfer galactose from UDP-galactose to lacto-*N*-triose II, thus generating lacto-N-neotetraose, is necessary. In a preferred embodiment, this production strain is further engineered by decreasing and/or diminishing the expression of the phosphofructokinase genes *pfkA* and/or *pfkB* and/or the glucose-6-phosphate dehydrogenase gene *zwf* and/or the glucose-6-phosphate isomerase gene *pgi.* This further genetic modification allows cultivation of the engineered production strain on a mixed-monosaccharide feedstock (e.g. hydrolyzed sucrose) as main carbon- and energy source, while preventing to hamper the strain's metabolism but increasing precursor supply (glucose and fructose-6-phosphate and glucose-6-phosphate) for lacto-*N*-neotetraose production by total fermentation. In an additional embodiment at least one of the *E. coli* genes *glmS, glmU, glmM, pgm, galU* and *galE* is overexpressed in order to foster the synthesis of UDP-GlcNAc and/or UDP-Gal.

In another embodiment, an *E. coli* strain possessing the genotype *lacY⁻*, *lacZ⁻*, *fuclK⁻*, *wcaJ⁻* is metabolically engineered to efficiently produce 2'-fucosyllactose by means of total fermentation using a mixed-monosaccharide feedstock (e.g. hydrolyzed sucrose) as main carbon- and energy source. Therefore, the expression of the glucokinase gene *glk* and/or the glucose dehydrogenase gene *gcd* as well as the activity of a phosphoenolpyruvate:glucose phosphotransferase dependent mechanism is decreased and/or abolished. In addition, phosphoenolpyruvate:sugar phosphotransferase independent (non-PEP-PTS) transporter gene is expressed or overexpressed in said *E. coli* strain. Furthermore, at least one of the E. *coli genes manA*, *manC*, *manB*, *gmd*, *wcaG*, *pgm*, *galU* and *galE* as well as a heterologous β-1,4-galactosyltransferase, capable to transfer galactose from UDP-galactose on glucose, thus generating lactose, and a α-1,2-fucosyltransferase, capable to transfer fucose from GDP-fucose to lactose, thus generating 2'-fucosyllactose, are expressed / overexpressed. In a preferred embodiment, this production strain is further engineered by decreasing and/or diminishing the expression of the phosphofructokinase genes *pfkA* and/or *pfkB* and/or the glucose-6-phosphate dehydrogenase gene *zwf* and/or the glucose-6-phosphate isomerase gene *pgi.* This further genetic modification allows cultivation of the engineered production strain on a mixed-monosaccharide feedstock (e.g. hydrolyzed sucrose) as main carbon- and energy source, while preventing to hamper the strain's metabolism but increasing precursor supply (glucose and fructose-6-phosphate and glucose-6-phosphate) for 2'-fucosyllactose production by total fermentation.

In another embodiment an *E. coli* strain possessing the genotype *lacY⁺*, *lacZ⁻*, *fuclK⁻*, *wcaJ⁻*, *manA⁻* is metabolically engineered as an efficient producer of 2'3-difucosyllactose using a mixed-monosaccharide feedstock (e.g. hydrolyzed sucrose) as main carbon- and energy source as well as lactose as acceptor substrate. Therefore, the expression of the genes exhibiting fructokinase activity (e.g. the *E*. *coli* K-12 *mak* gene) and the activity of a phosphoenolpyruvate:fructose phosphotransferase dependent mechanism is decreased and/or abolished. This cell is further genetically engineered by the expression/overexpression of at least one gene encoding for a phosphoenolpyruvate:fructose phosphotransferase independent (non-PEP-PTS) transporter, enabling fructose transfer into the engineered cell, a mannose isomerase, capable of transforming fructose to mannose (e.g. *E. coli* BL21 *yihS, Agrobacterium radiobacter* M-1 *manl),* a mannokinase (e.g. *Prevotella bryantii* β₁4 *manK, Arthrobacter sp.* Strain KM *manK*)*,* capable of activating mannose to mannose-6-phosphate, as well as for at least one heterologous fucosyltransferase exhibiting an α-1,2- and/or an α-1,3-fucosyltransferase activity, capable of transferring fucose from GDP-fucose to lactose and/or 2'-fucosyllactose and/or 3-fucosyllactose, thus generating 2'3-difucosyllactose. These further genetic modifications allow cultivation of the engineered production strain on a mixed-monosaccharide feedstock (e.g. hydrolyzed sucrose) as main carbon- and energy source, while preventing to hamper the strain's metabolism but increasing precursor supply (mannose/mannose-6-phosphate) for 2'3-difucosyllactose production. In an additional embodiment the overexpression of at least one of the *E*. *coli* genes *manC, manB, gmd* and *wcaG* is overexpressed in order to foster the synthesis of GDP-Fuc.

Non-limiting examples for suitable proteins exhibiting mannose isomerase (Manl) activity or mannokinase (ManK) activity can be found in the literature (Hirose et al., Biosci Biotechnol Biochem. 2001 Mar;65(3):658-61.; Hirose et al., Biotechnol Lett. 2003 Feb;25(4):349-52.; Patel et al., Appl Environ Microbiol. 2011 May;77(10):3343-50.; Hu et al., Int J Biol Macromol. 2016 Aug;89:328-35.; Huang et al., Appl Microbiol Biotechnol. 2018 Mar;102(5):2051-2062.; Mukai et al., Appl Environ Microbiol. 2003 Jul;69(7):3849-57.; Fields and Russel, Microbiology. 2001 Apr;147(Pt 4):1035-43.; Kroschewski et al., Mol Biochem Parasitol. 2000 Jan 5;105(1):71-80.).

In another embodiment an *E. coli* strain possessing the genotype *nagAB⁻, fuclK⁻, wcaJ⁻* is metabolically engineered as an efficient producer of H antigen type I (HA-T I) by means of total fermentation using a mixed-monosaccharide feedstock (e.g. a mixture of hydrolyzed sucrose and hydrolyzed lactose) as main carbon- and energy source. Therefore, the production strain is genetically engineered by decreasing and/or diminishing the import of glucose through a phosphoenolpyruvate:sugar phosphotransferase dependent mechanism, while expressing/overexpressing at least one gene encoding for a non-PEP-PTS transporter as well as a monosaccharide kinase, enabling glucose and/or galactose transfer into the engineered cell as well as activating these monosaccharides into a phosphorylated form, respectively. Furthermore, at least one gene encoding for a glucosamine-6-phosphate acetyltransferase, capable to transfer acetate from acetyl-CoA to glucosamine-6-phosphate, thus generating *N*-acetylglucosamine-6-phosphate, a HAD-like sugar phosphatase, capable to dephosphorylate *N*-acetylglucosamine-6-phosphate, thus generating *N*-acetylglucosamine, a heterologous β-1,3-galactosyl-transferase, capable to transfer galactose from UDP-galactose on *N*-acetylglucosamine, thus generating lacto-*N*-biose, and an α-1,2-fucosyltransferase, capable to transfer fucose from GDP-fucose to lacto-*N*-biose, thus generating H antigen type I, are expressed/overexpressed. In a preferred embodiment, this production strain is further genetically engineered by decreasing and/or diminishing the expression of the phosphofructokinase genes *pfkA* and/or *pfkB.* These genetic modifications allow cultivation of the engineered production strain on a mixed-monosaccharide feedstock (e.g. a mixture of hydrolyzed sucrose and hydrolyzed lactose) as main carbon- and energy source, while preventing to hamper the strain's metabolism but increasing precursor supply (*N*-acetylglucosamine, fructose-6-phosphate and galactose-1-phosphate) for H antigen type I production. In an additional embodiment at least one of the *E. coli* genes *glmS*, *galT*, *galE*, *manC*, *manB*, *gmd* and *wcaG* is overexpressed in order to foster the synthesis of GlcNAc and/or UDP-Gal and/or GDP-Fuc.

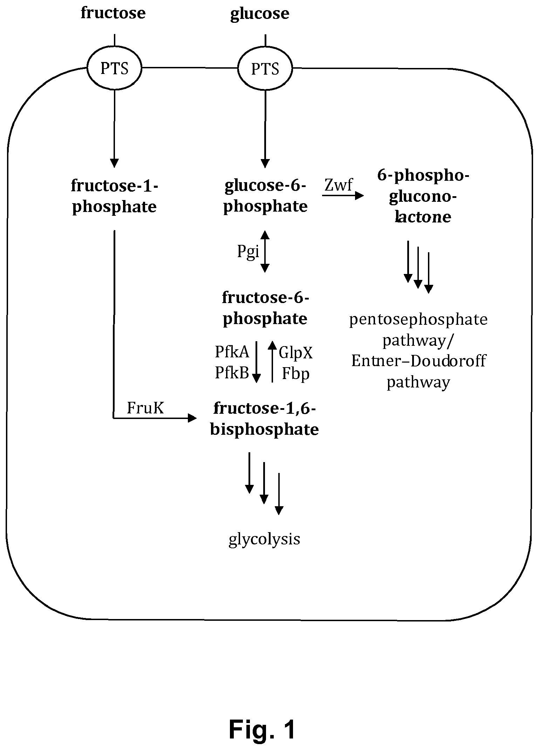

Referring to Fig. 1, an exemplary natural microbial cell is schematically shown. Said microbial cell is capable of consuming a mixed feedstock consisting of glucose and fructose, leading to a minute intracellular pool of glucose-6-phosphate and fructose-6-phosphate. The microbial cell expresses polynucleotides encoding phosphoenolpyruvate:sugar phosphotransferase systems for glucose and fructose (PTS) import into the cell. The reaction products primarily enter the glycolysis.

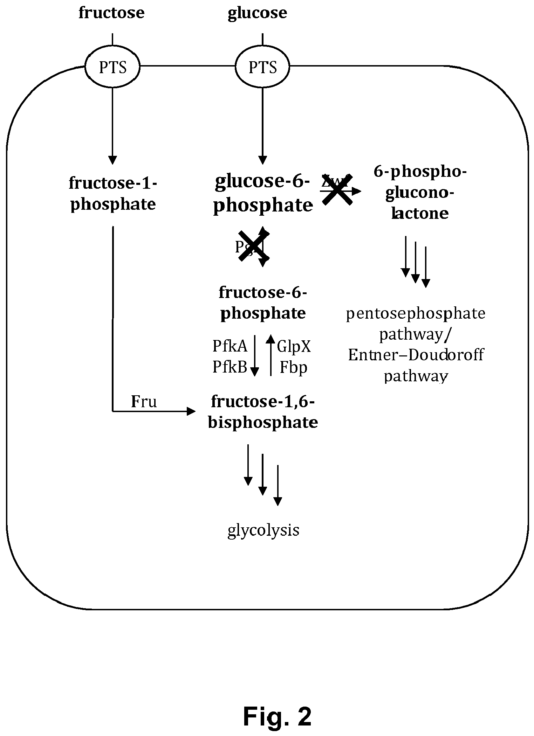

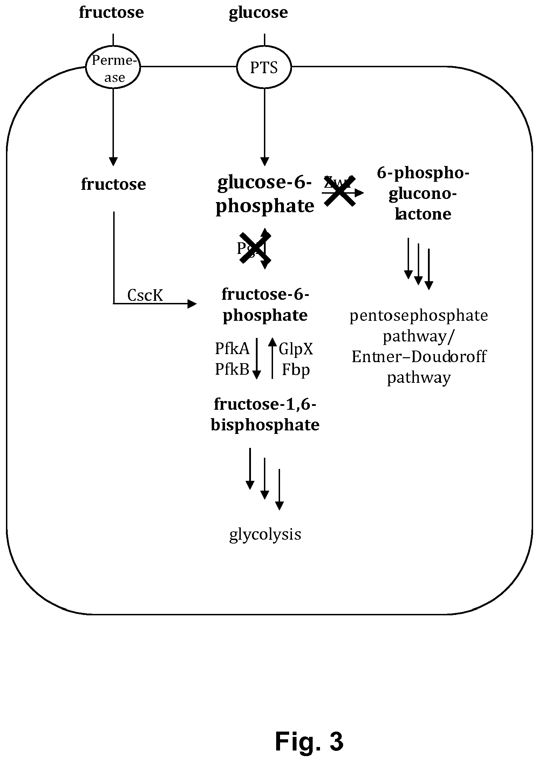

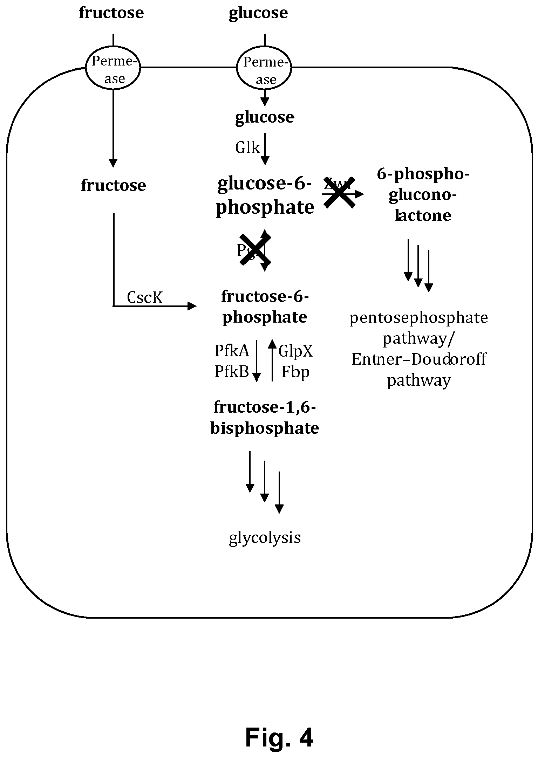

Fig. 2 displays schematically an exemplary microbial cell of the invention being capable of supplying glucose-6-phosphate to a high degree when cultivated on a mixed feedstock consisting of glucose and fructose. As expression of the glucose-6-phosphate isomerase Pgi and/or the glucose-6-phosphate dehydrogenase Zwf have been decreased or diminished by deletion, functional inactivation or silencing of the *pgi* and/or *zwf* gene(s), any glucose-6-phosphate generated by glucose import into the cell may be utilized by the microbial cell for generating UDP-Glc and/or UDP-Gal. In a variant of the microbial cell (Fig. 3), the cell's phosphoenolpyruvate:sugar phosphotransferase system(s) for fructose has been disabled, e.g. by deletion of the *fruA* gene. Instead, fructose enters the cell via a phosphoenolpyruvate:sugar phosphotransferase independent (non-PEP-PTS) transporter getting activated by a fructose kinase (e.g. *E. coli W* CscK) to fructose-6-phosphate. In a variant of the microbial cell (Fig. 4), the cell's phosphoenolpyruvate:sugar phosphotransferase system(s) for glucose has also been disabled, e.g. by deletion of the *ptsG* gene. Instead, glucose enters the cell via a phosphoenolpyruvate:sugar phosphotransferase independent (non-PEP-PTS) transporter getting activated by a glucose kinase (e.g. *E. coli* K-12 Glk) to glucose-6-phosphate.

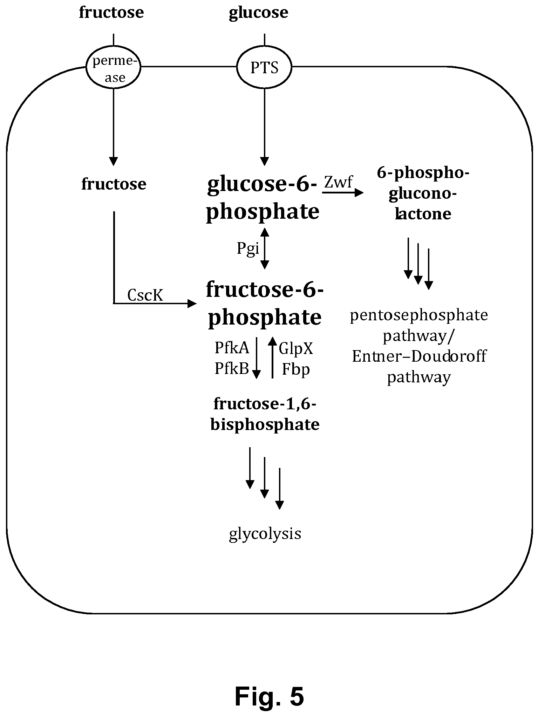

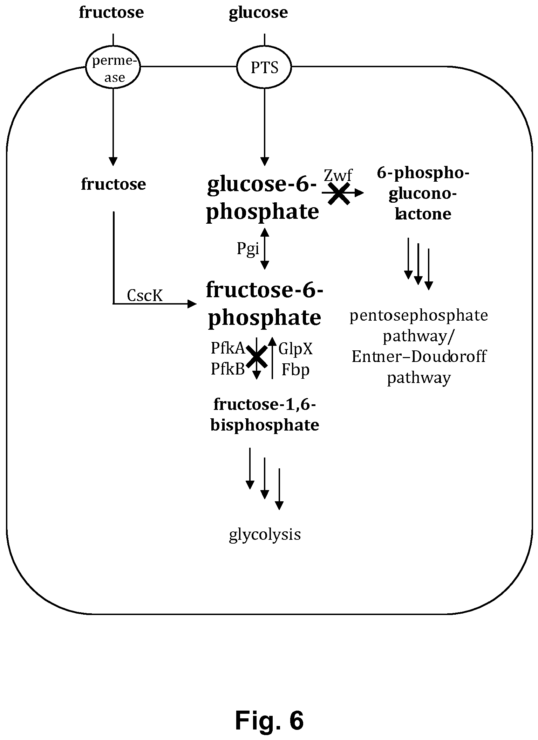

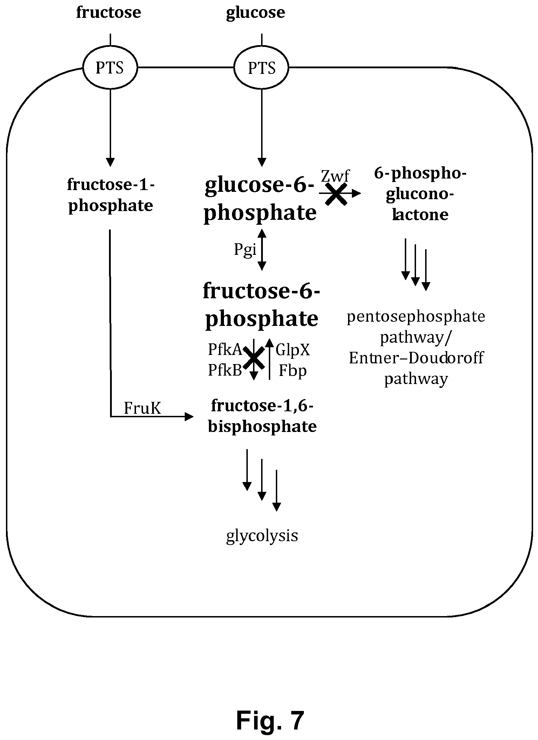

Fig. 5 displays schematically an exemplary microbial cell of the invention being capable of supplying glucose-6-phosphate and fructose-6-phosphate to a higher degree when cultivated on a mixed feedstock consisting of glucose and fructose. The cell's phosphoenolpyruvate:sugar phosphotransferase system(s) for fructose has been disabled, e.g. by deletion of the *fruA* gene. Instead, fructose enters the cell via a phosphoenolpyruvate:sugar phosphotransferase independent (non-PEP-PTS) transporter getting activated by a fructose kinase (e.g. *E*. *coli W* CscK) to fructose-6-phosphate. In a variant of the microbial cell (Fig. 6), the expression of the phosphofructokinase(s) Pfk and/or the glucose-6-phosphate dehydrogenase Zwf have been decreased or diminished by deletion, functional inactivation or silencing of the *pfkA* and/or *pfkB* and/or *zwf* gene(s), yielding increased availability of glucose-6-phosphate and fructose-6-phosphate, which may be utilized by the microbial cell for generating UDP-Gal and/or GDP-Fuc and/or UDP-GlcNAc and/or CMP-Neu5Ac. In a variant of the microbial cell (Fig. 7), the cell expresses polynucleotides encoding phosphoenolpyruvate:sugar phosphotransferase systems for glucose and fructose (PTS) import into the cell. The expression of the phosphofructokinase(s) Pfk and/or the glucose-6-phosphate dehydrogenase Zwf have been decreased or diminished by deletion, functional inactivation or silencing of the *pfkA* and/or *pfkB* and/or *zwf* gene(s), yielding increased availability of glucose-6-phosphate and fructose-6-phosphate.

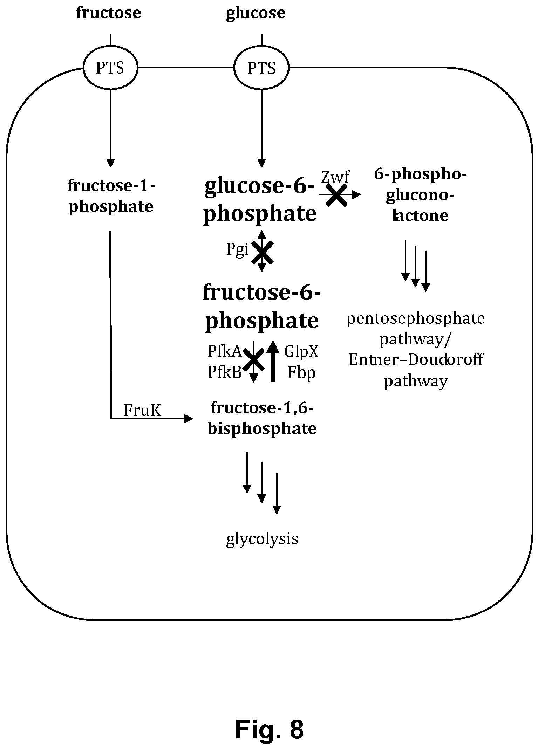

Fig. 8 displays schematically an exemplary microbial cell of the invention being capable of supplying glucose-6-phosphate and fructose-6-phosphate to a higher degree when cultivated on a mixed feedstock consisting of glucose and fructose. The cell expresses polynucleotides encoding phosphoenolpyruvate:sugar phosphotransferase systems for glucose and fructose (PTS) import into the cell. The expression of the phosphofructokinase(s) Pfk and/or the glucose-6-phosphate isomerase Pgi and/or the glucose-6-phosphate dehydrogenase Zwf have been decreased or diminished by deletion, functional inactivation or silencing of the *pfkA* and/or *pfkB* and/or *pgi* and/or *zwf* gene(s). Along with an overexpression of a fructose-1,6-bisphosphatase (e.g. *glpX, fbp)* an increased availability of glucose-6-phosphate and fructose-6-phosphate is generated and may be utilized by the microbial cell for generating UDP-Gal and/or GDP-Fuc and/or UDP-GlcNAc and/or CMP-Neu5Ac.

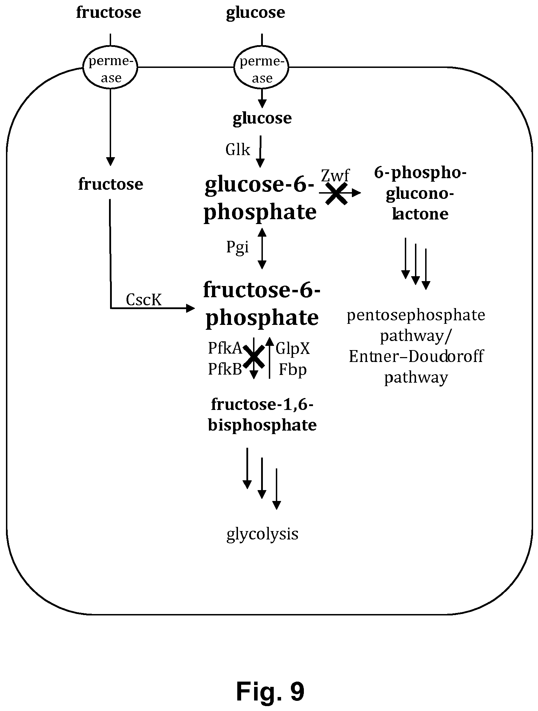

Fig. 9 displays schematically an exemplary microbial cell of the invention being capable of generating high availability of glucose-6-phosphate and fructose-6-phosphate and phosphoenolpyruvate when cultivated on a mixed feedstock consisting of glucose and fructose. The cell's phosphoenolpyruvate:sugar phosphotransferase systems for glucose and fructose have been disabled, e.g. by deletion of the *ptsG* and *fruA* genes, respectively. Instead, fructose and glucose enter the cell via a phosphoenolpyruvate:sugar phosphotransferase independent (non-PEP-PTS) transporter getting activated by a fructose kinase (e.g. *E. coli* W CscK) and glucose kinase (e.g. *E. coli* K-12 Glk), to fructose-6-phosphate and glucose-6-phosphate, respectively. Moreover, PEP consumption by the phosphoenolpyruvate:sugar phosphotransferase systems is circumvented. Along with the decreased and/or diminished expression of the phosphofructokinase(s) Pfk and/or the glucose-6-phosphate dehydrogenase Zwf, realized by deletion, functional inactivation or silencing of the *pfkA* and/or *pfkB* and/or *zwf* gene(s), increased availability of glucose-6-phosphate and fructose-6-phosphate and phosphorenolpyruvate is achieved. This may be utilized by the microbial cell for generating UDP-Gal and/or GDP-Fuc and/or UDP-GlcNAc and/or CMP-Neu5Ac.

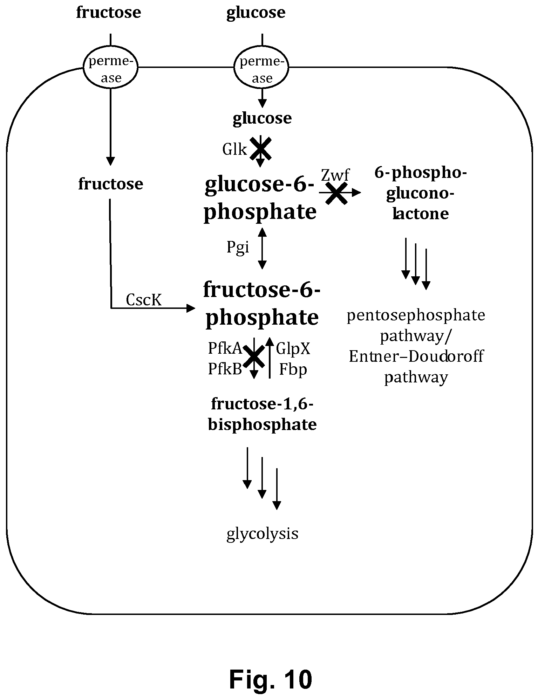

Fig. 10 displays schematically another exemplary microbial cell of the invention capable of generating increased availability of free glucose and glucose-6-phosphate and fructose-6-phosphate when cultivated on a mixed feedstock consisting of glucose and fructose. The cell's phosphoenolpyruvate:sugar phosphotransferase systems for glucose and fructose have been disabled, e.g. by deletion of the *ptsG* and *fruA* genes, respectively. Instead, fructose and glucose enter the cell via a phosphoenolpyruvate:sugar phosphotransferase independent (non-PEP-PTS) transporter. Along with the deletion of the *glk* gene, the microbial cell has been genetically engineered such that free glucose monomer becomes available in the cell. Instead, fructose gets activated by a fructose kinase (e.g. *E*. *coli* W CscK) to fructose-6-phosphate. The expression of the phosphofructokinase(s) Pfk and/or the glucose-6-phosphate dehydrogenase Zwf have been decreased or diminished by deletion, functional inactivation or silencing of the *pfkA* and/or *pfkB* and/or *zwf* gene(s). Overall, this is yielding an improved intracellular supply of free glucose and glucose-6-phosphate and fructose-6-phosphate. Glucose becomes available as substrate for various glycosylation reactions, whereas glucose-6-phosphate and fructose-6-phosphate may be utilized by the microbial cell for generating UDP-Gal and/or GDP-Fuc and/or UDP-GlcNAc and/or CMP-Neu5Ac. Additionally, PEP consumption by the phosphoenolpyruvate:sugar phosphotransferase systems is circumvented.

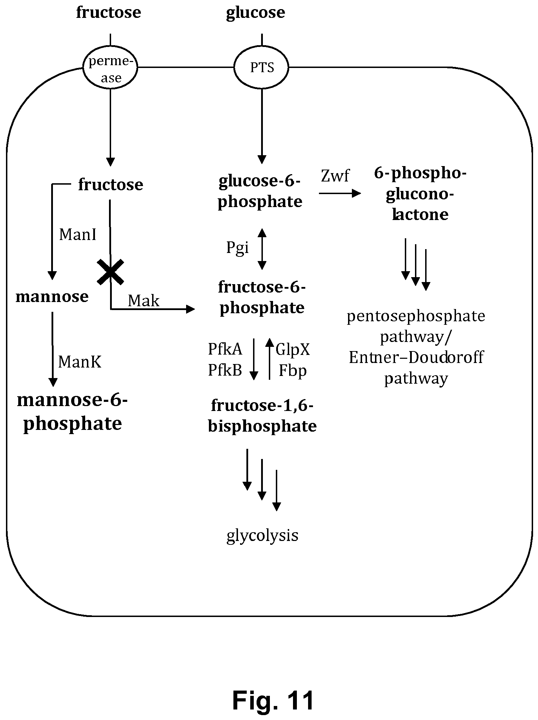

Fig. 11 displays schematically another exemplary microbial cell of the invention capable of achieving an increased intracellular availability of free mannose and/or mannose-6-phosphate when cultivated on a mixed feedstock consisting of glucose and fructose. The cell's phosphoenolpyruvate:sugar phosphotransferase systems for fructose have been disabled, e.g. by deletion of the *fruA* gene and/or the *manXYZ* genes. Instead, fructose enters the cell via a sugar permease and/or channel. Along with the deletion of genes exhibiting fructose kinase activity (e.g. mak) and/or mannose-6-phosphate isomerase activity (e.g. *manA*) as well as the expression/overexpression of a suitable mannose isomerase (Manl) and mannokinase (ManK), the microbial cell has been genetically engineered such that mannose-6-phosphate becomes available in the cell. This may be utilized by the microbial cell for generating GDP-Man and/or GDP-Fuc.

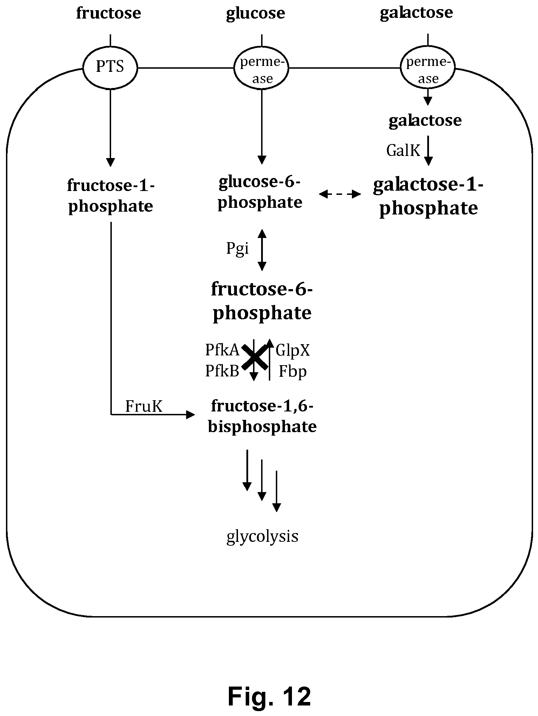

Fig. 12 displays schematically another exemplary microbial cell of the invention capable of generating a higher intracellular availability of free fructose-6-phosphate and/or galactose-1-phosphate when cultivated on a mixed feedstock consisting of glucose and fructose and galactose. The cell's phosphoenolpyruvate:sugar phosphotransferase systems for glucose has been disabled, e.g. by deletion of the *ptsG* gene. Instead, glucose enters the cell via a phosphoenolpyruvate:sugar phosphotransferase independent (non-PEP-PTS) transporter. The expression of the phosphofructokinase(s) Pfk have been decreased or diminished by deletion, functional inactivation or silencing of the *pfkA* and/or *pfkB* gene(s). Along with the expression/overexpression of a suitable non-PEP-PTS transporter as well as of a monosaccharide kinase, enabling galactose transfer into the engineered cell as well as its activation to galactose-1-phosphate, respectively, the microbial cell has been genetically engineered such that fructose-6-phosphate and galactose-1-phosphate become available in the cell. These may be utilized by the microbial cell for generating GlcNAc and/or GDP-Fuc and/or UDP-Gal and/or CMP-Neu5Ac.

According to the fourth aspect, provided is the use of the desired carbohydrates in a pharmaceutical and/or nutritional composition, wherein the carbohydrate of interest is preferably produced by a method or genetically engineered host cell according to the invention.

### Examples

### Example 1 - Preparation of a mixed monosaccharide feedstock

A 50 % (w/v) sucrose solution was prepared by dissolving 500 g of sucrose in water. The final volume of the solution was 1 litre. At a temperature of 30°C to 35°C the pH was adjusted by using 96 % (v/v) sulfuric acid. Afterwards, the solution was sterilized in a vertical autoclave (Systec VX-65, Linden, Germany) at 121°C for 45 minutes. Samples were taken before and after heat sterilization and kept frozen prior to analysis by high performance liquid chromatography (HPLC). HPLC was carried out using a RID-10A refractive index detector (Shimadzu, Germany) and a Waters XBridge Amide Column 3.5 µm (250 x 4.6 mm) (Eschborn, Germany) connected to a Shimadzu HPLC system. Isocratic elution was carried out with 30% solvent A (50% (v/v) acetonitrile in double distilled water, 0.1% (v/v) NH4OH) and 70% solvent B (80% (v/v) acetonitrile in double distilled water, 0.1% (v/v) NH4OH) at 35°C and at a flow rate of 1.4 mL min-1. Samples were cleared by solid phase extraction on an ion exchange matrix (Strata ABW, Phenomenex). Ten microliters of the sample (dilution of 1:5) was applied to the column. Finally, the relative amount of detected sugars was determined. As depicted in table 2, the sucrose conversion into the monosaccharides glucose and fructose increased with decreasing pH values of the solutions prior to heat treatment. Full sucrose cleavage could be observed at pH values ≤ 3.50 when acidification was carried out with sulfuric acid.

**Table 2: Relative amount of sugars detected in pH adjusted 50 % (w/v) sucrose solution before and after heat sterilization. The pH adjustment was carried out using 96 % (v/v) sulfuric acid. Depicted is the percental amount of sugars (area under the curve; AUC) detected by HPLC.**

| pH | Relative composition [%] | | |
|---|---|---|---|
| | Sucrose | Glucose | Fructose |
| **before heat sterilization** | | | |
| 3.50 - 7.10 | 100 | - | - |

| **after heat sterilization** | | | |
|---|---|---|---|
| 7.10 (not acidified) | 100 | - | - |
| pH 5.50 | 87.55 | 6.30 | 6.15 |
| pH 5.05 | 68.25 | 16.62 | 15.13 |
| pH 3.87 | 13.90 | 45.05 | 41.06 |
| pH 3.50 | - | 51.68 | 48.32 |

### Example 2 - Feedstock-dependent growth of various gene deletion strains

The growth behavior of an *E. coli* BL21(DE3) strain (wild type) as well as the mutated strains *E*. *coli pfka⁻* (*ΔpfkA*), *E. coli pfkB⁻* (*ΔpfkB*), *E. coli pfkA⁻ pfkB⁻* (*ΔpfkA ΔpfkA*) was compared. Genomic deletions were performed according to the method of Datsenko and Wanner (Proc. Natl. Acad. Sci. USA 97:6640-6645 (2000)). All strains were cultivated at 30°C in 100 mL-shake flasks with 20 mL mineral salts medium, containing 7 g·L⁻¹ NH₄H₂PO₄, 7 g.L⁻¹ K₂HPO₄, 2 g.L⁻¹ KOH, 0.3 g.L⁻¹ citric acid, 2 g.L⁻¹ MgSO₄ × 7·H₂O and 0.015 g·L-¹ CaCl₂ × 6·H₂O, supplemented with 1 mL·L⁻¹ trace element solution (54.4 g.L⁻¹ ammonium ferric citrate, 9.8 g.L⁻¹ MnCl₂ × 4·H₂O, 1.6 g.L⁻¹ CoCl₂ × 6·H₂O, 1 g.L⁻¹ CuCl₂ × 2·H₂O, 1.9 g.L⁻¹ H₃BO₃, 9 g.L⁻¹ ZnSO₄ × 7·H₂O, 1.1 g.L⁻¹ Na₂MoO₄ × 2·H₂O, 1.5 g.L⁻¹ Na₂SeO₃, 1.5 g.L⁻¹ NiSO₄ × 6·H₂O) and containing either 2 % (m/v) glucose (A) or 1 % (w/v) glucose/1 % (w/v) fructose (B) as carbon source. Cultures were inoculated to OD 0.1 and growth development was monitored over 26 hours by OD₆₀₀ measurement. As shown in Fig. 2, *E. coli pfka⁻ pfkB⁻* hardly showed growth when glucose was provided as sole carbon- and energy source, whereas its growth was indistinguishable from the wild type strain as well as the single deletion mutants when the mixed monosaccharide feedstock was available.

### Example 3 - Production of 2'-fucosyllactose by an engineered E. coli strain

An *E. coli* BL21 (DE3) strain exhibiting the genotype *lacY⁺*, *lacZ⁻*, *fuclK⁻*, *wcaJ⁻*, *pfkA⁻*, was further genetically engineered by overexpressing enzymes for the de novo synthesis of GDP-Fucose (ManB, ManC, Gmd, WcaG), the 2'-fucosyltransferase gene *wbgL* from *E*. *coli:*O126*,* the sugar efflux transporter yberc0001_9420 from *Yersinia bercovieri* ATCC 43970 and the csc-gene cluster of *E. coli* W (acc. no. CP002185.1) comprising the genes for sucrose permease, fructokinase, sucrose hydrolase as well as a transcriptional repressor (genes *cscB*, *cscK*, *cscA*, and *cscR*, respectively), enabling the strain to grow on sucrose as sole carbon source. Genomic deletions were performed according to the method of Datsenko and Wanner (Proc. Natl. Acad. Sci. USA 97:6640-6645 (2000)). Genomic integration of heterologous genes was performed by transposition. Either the EZ-Tn5TM transposase (Epicentre, USA) was used to integrate linear DNA-fragments or the hyperactive C9-mutant of the mariner transposase Himar1 (Proc. Natl. Acad. Sci. 1999, USA 96:11428-11433) was employed for transposition. The genes were codon-optimized for expression in *E. coli* and prepared synthetically by GenScript cooperation. The resulting strain was cultivated at 30°C in 100 mL-shake flasks with 20 mL mineral salts medium, containing 3 g/L KH₂PO₄, 12 g/L K₂HPO₄, 5 g/L (NH₄)₂SO₄, 0.3 g/L citric acid, 2 g/L MgSO₄ × 7H₂O, 0.1 g/L NaCl and 0.015 g/L CaCl₂ × 6H₂O with 1 mL/L trace element solution (54.4 g/L ammonium ferric citrate, 9.8 g/L MnCl₂ × 4H₂O, 1.6 g/L CoCl₂ × 6H₂O, 1 g/L CuCl₂ × 2H₂O, 1.9 g/L H₃BO₃, 9 g/L ZnSO₄ × 7H₂O, 1.1 g/L Na₂MoO₄ 2H₂O, 1.5 g/L Na₂SeO₃, 1.5 g/L NiSO₄ × 6H₂O), and containing 2 % (v/v) glycerol, 2 % (m/v) sterile-filtered sucrose or 2 % sucrose hydrolysate as carbon source. Additionally, 15 mM lactose were added, yielding as acceptor substrate for 2'-fucosyllactose production. Cultures were inoculated to OD 0.1 and cultivation was discontinued after 26 hours. For quantifying 2'-FL in the culture broth, HPLC analyses were performed using a refractive index detector (RID-10A) (Shimadzu, Germany) and a Waters XBridge Amide Column 3.5µm (250 x 4.6mm) (Eschborn, Germany) connected to an HPLC system (Shimadzu, Germany). Elution was performed isocratically with 30% A: 50% (v/v) ACN in ddH₂O, 0.1% (v/v) NH₄OH and 70% B: 80% (v/v) ACN in ddH₂O, 0.1% (v/v) NHₐOH (v/v) as eluent at 35°C and at a flow rate of 1.4 ml·min⁻¹. The culture supernatant was sterile filtered (0.22 µm pore size) and cleared by solid phase extraction on an ion exchange matrix (Strata ABW, Phenomenex). 10 µl of the samples were applied to the column, and the 2'-fucosyllactose concentration was calculated according to a standard curve. The productivity of the engineered strain during growth on glycerol was set to 100 %. As depicted in table 3, 2'-FL production was highest when sucrose hydrolysate was provided as carbon- and energy source.

**Table 3: Relative 2'-FL production of the E. coli strain described in example 3 during cultivation on glycerol, sucrose or sucrose hydrolysate as carbon- and energy source. The productivity on glycerol was set to 100 %.**

| | Glycerol | Sucrose | Sucrose hydrolysate |
|---|---|---|---|
| Relative 2'-FL production | 100 % | 199 % | 482 % |

### Example 4 - Total fermentation of 2'-fucosyllactose by an engineered E. coli strain during growth on a mixed-monosaccharide feedstock

An *E*. *coli* BL21 (DE3) strain exhibiting the genotype *pfkA⁻*, *lacZ⁻*, *fuclK⁻*, *wcaJ⁻*, *glk⁻*, *gcd⁻*, *ptsG⁻* was further genetically engineered by overexpressing enzymes for the de novo synthesis of GDP-Fucose (ManB, ManC, Gmd, WcaG), the 2'-fucosyltransferase gene *wbgL* from *E*. *coli:*O126, the sugar efflux transporter gene *yberc0001_9420* from *Yersinia bercovieri* ATCC 43970, the glucose facilitator gene *glf* from *Zymomonas mobilis*, the β-1,4-galactosyltransferase gene *galTpm1141* from *Pasteurella multocida* (GenBank: AEC04686) as well as the *E*. *coli* genes *galE* and *pgm,* encoding a UDP-glucose 4-epimerase and a phosphoglucomutase, respectively. Genomic deletions were performed according to the method of Datsenko and Wanner (Proc. Natl. Acad. Sci. USA 97:6640-6645 (2000)). Genomic integration of heterologous genes was performed by transposition. Either the EZ-Tn5TM transposase (Epicentre, USA) was used to integrate linear DNA-fragments or the hyperactive C9-mutant of the mariner transposase Himar1 (Proc. Natl. Acad. Sci. 1999, USA 96:11428-11433) was employed for transposition. The genes were codon-optimized for expression in *E. coli* and prepared synthetically by GenScript cooperation.

The resulting *E. coli* strain was cultivated at 30°C in a 3 L fermenter (New Bruns-wick, Edison, USA) starting with 1000 mL mineral salts medium containing 7 g.L⁻¹ NH₄H₂PO₄, 7 g.L⁻¹ K₂HPO₄, 2 g.L⁻¹ KOH, 0.3 g.L⁻¹ citric acid, 2 g.L⁻¹ MgSO₄ × 7·H₂O and 0.015 g·L⁻¹ CaCl₂ × 6·H₂O, supplemented with 1 mL·L⁻¹ trace element solution (54.4 g.L⁻¹ ammonium ferric citrate, 9.8 g.L⁻¹ MnCl₂ × 4·H₂O, 1.6 g.L⁻¹ CoCl₂ × 6·H₂O, 1 g.L⁻¹ CuCl₂ × 2·H₂O, 1.9 g.L⁻¹ H₃BO₃, 9 g.L⁻¹ ZnSO₄ × 7·H₂O, 1.1 g.L⁻¹ Na₂MoO₄ × 2·H₂O, 1.5 g.L⁻¹ Na₂SeO₃, 1.5 g.L⁻¹ NiSO₄ × 6·H₂O) and containing 2 % (m/v) hydrolyzed sucrose as carbon source. Cultivation was started with the addition of a 2.5 % (v/v) inoculum from a pre-culture grown in the same medium. The end of the batch phase was characterized by a rise in the dissolved oxygen level. A carbon feed consisting of fully hydrolyzed sucrose, supplemented with 2 g.L⁻¹ MgSO₄ × 7·H₂O, 0.015 g·L-¹CaCl₂ × 6·H₂O and 1 mL·L⁻¹ trace element solution, was applied instantaneously after leaving the batch phase. A feeding rate of 12.0 - 15.0 mL·L⁻¹·h⁻¹ was applied, referring to the starting volume. Aeration was maintained at 3 L·min⁻1. Dissolved oxygen was maintained at 20-30 % saturation by controlling the rate of agitation. The pH was maintained at 6.7 by adding 25 % ammonia solution. Cultivation lasted for 86 hours and yielded substantial amounts of 2'-FL in the culture supernatant.

## Claims

1. A genetically engineered microbial cell for the production of a carbohydrate of interest, wherein the microbial cell possesses an increased intracellular availability of at least one sugar phosphate as compared to the wild-type cell, and produces the carbohydrate of interest when being cultivated in a culture medium comprising a mixed monosaccharide feedstock as main carbon and energy source, wherein said mixed monosaccharide feedstock consists of glucose and at least one additional monosaccharide selected from the group consisting of fructose and galactose.

2. The genetically engineered microbial cell according to claim 1, wherein the genetically engineered microbial cell further comprises an UDP-galactose biosynthesis pathway for intracellular formation of UDP-galactose.

3. The genetically engineered microbial cell according to claim 1 or 2, wherein the genetically engineered microbial cell further comprises a GDP-fucose biosynthesis pathway for intracellular formation of GDP-L-fucose.

4. The genetically engineered microbial cell according to any one of claims 1 to 3, wherein the genetically engineered microbial cell further comprises an UDP-N-acetylglucosamine biosynthesis pathway for intracellular formation of UDP-N-acetylglucosamine.

5. The genetically engineered microbial cell according to any one of claims 1 to 4, wherein the genetically engineered microbial cell further comprises an CMP-N-acetylneuraminic acid/CMP-sialic acid biosynthesis pathway for intracellular formation of CMP-N-acetylneuraminic acid.

6. The genetically engineered microbial cell according to any one of claims 1 to 5, wherein the genetically engineered microbial cell further comprises at least one glycosyltransferase.

7. The genetically engineered microbial cell according to any one of claims 1 to 6, wherein the genetically engineered microbial cell further comprises an enhanced synthesis of phosphoenolpyruvate.

8. The genetically engineered microbial cell according to any one of claims 1 to 7, wherein the genetically engineered microbial cell further comprises at least one monosaccharide transporter for translocating a monosaccharide from the medium into the cytoplasm of the microbial cell.

9. The genetically engineered microbial cell according to any one of claims 1 to 8, wherein the genetically engineered microbial cell further comprises a decreased or diminished expression of at least one gene encoding for a protein competing with the biosynthesis of the desired carbohydrate and/or decreased or diminished activity of at least one protein competing with the biosynthesis of the carbohydrate of interest.

10. The genetically engineered microbial cell according to any one of claims 1 to 9, wherein the protein leading to a consumption of said intracellular sugar phosphate within said engineered microorganism is selected from the group consisting of phosphofructokinase, glucose-6-phosphate isomerase, glucose-6-phosphate dehydrogenase, components of a phosphoenolpyruvate:sugar phosphotransferase system

11. The genetically engineered microbial cell according to any one of claims 1 to 10, wherein the genetically engineered microbial cell further comprises at least one exporter protein or permease exporting the carbohydrate of interest from the cell.

12. Use of a genetically engineered microbial cell according to any one of claims 1 to N for the production of a carbohydrate of interest.

13. A method for the fermentative production of a carbohydrate of interest, the method comprising:
a) providing a genetically engineered microorganism capable of producing a desired carbohydrate, wherein said microorganism is exhibiting an increased intracellular availability of at least one sugar phosphate due to a decreased and/or diminished expression and/or activity of at least one protein leading to a consumption of said intracellular sugar phosphate within said engineered microorganism;
b) cultivating said genetically engineered microorganism in a culture medium permissive for the production of said desired carbohydrate, wherein the main carbon source is a monosaccharide mixture consisting of glucose and at least a second monosaccharide of the group of fructose and galactose; and
c) recovering said carbohydrate of interest.

14. The method according to claim 13, wherein the carbohydrate of interest is selected from the group consisting of mannose, fucose, N-acetylglucosamine, *N*-acetylmannosamine, *N*-acetylneuraminic acid, lacto-N-biose, *N*-acetyllactosamine, 2'-fucosyllactose, 3-fucosyllactose, 2',3-difucosyllactose, blood group H antigen type I, blood group H antigen type II, blood group antigen Lewis^{a}, blood group antigen Lewis^{b}, blood group antigen Lewis^{x}, blood group antigen Lewis^{y}, blood group antigen Sialyl-Lewis^{x}, lacto-N-triose II, lacto-*N*-tetraose, lacto-*N*-neotetraose, lacto-*N*-fucopentaose I, lacto-*N*-neofucopentaose I, lacto-*N*-fucopentaose II, lacto-*N*-fucopentaose III, lacto-*N*-fucopentaose V, lacto-*N*-neofucopentaose V, lacto-*N*-difucohexaose I, lacto-*N*-difucohexaose II, 6'-galactosyllactose, 3'-galactosyllactose, lacto-N-hexaose, lacto-*N*-neohexaose, *para*-lacto-*N*-hexaose, para-Lacto-N-neohexaose, difucosyl-lacto-*N*-neohexaose, 3'-sialyllactose, 6'-sialyllactose, 3'-sialyl-*N*-acetyllactosamine, 6'-sialyl-N-acetyllactosamine, lacto-N-sialylpentaose a, lacto-*N*-sialylpentaose b, lacto-*N*-sialylpentaose c, fucosyl-lacto-*N*-sialylpentaose a , fucosyl-lacto-*N*-sialylpentaose b, fucosyl-lacto-*N-*sialylpentaose c, disialyl-lacto-*N*-tetraose, disialyl-lacto-*N*-fucopentaose, 3-fucosyl-3'-sialyllactose, 3-fucosyl-6'-sialyllactose, lacto-*N*-neodifucohexaose I.

15. Use of a carbohydrate of interest being produced by a microbial cell according to any one of claims 1 to 11 and/or a method according to any one of claims 13 to 14 for the manufacturing of a pharmaceutical and/or nutritional composition.
